# EUROPEAN PATENT APPLICATION

(11) **EP 4 552 673 A1**
(43) Date of publication of application: **14.05.2025**
(21) Application number: 23208238.8
(22) Date of filing: 07.11.2023
(51) Int. Cl.: A61M 5/20, A61M 5/32, A61M 5/24, A61M 5/31, A61M 5/315

(54) **INJECTION DEVICE WITH REDUCED AMOUNT OF PARTS**

(71) Applicant: Ypsomed AG, 3401 Burgdorf (CH)
(72) Inventor: Loser, Peter, 3037 Herrenschwanden (CH); Tschirren, Markus, 3400 Burgdorf (CH); Glauser, Oliver, 3008 Bern (CH); Zumstein, Dominik, 3014 Bern (CH); Bosshard, Simon Martin, 3324 Hindelbank (CH); Schrul, Christian, 3414 Oberburg (CH)
(74) Representative: Kleiner, Stefan

(57) **Abstract**

The invention relates to an injection device (1) comprising a pre-assembled drive unit (3) connected to a pre-assembled syringe unit (2). The pre-assembled syringe unit (2) comprises a syringe holder (30) and a needle cover (20) and wherein the pre-assembled drive unit (3) comprises a plunger rod (40), a spring member (90) adapted bias the plunger rod (40) in a dispensing direction, a supporting member (60) and a first and a second retaining element (67, 65), wherein each retaining element (67, 65) can be in a holding position in which the first or second retaining element holds the biased plunger rod (40) in place relative to the supporting member (60) and in a release position in which the first or second retaining element (67, 65) does not hold the biased plunger rod (40) in place.

## Description

### FIELD OF THE INVENTION

The present invention relates to drug delivery devices or medicament delivery devices for injecting, delivering, administering, infusing or dispensing substances and/or liquids such as insulin, hormone preparations or vaccines. It departs from an Injection device comprising a pre-assembled drive unit connected to a pre-assembled syringe unit.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors comprising prefilled syringes. Autoinjectors usually comprise a body for housing the syringe as well as an automatic drive mechanism to move the plunger of the syringe upon actuation of the autoinjector. The drive mechanism of the autoinjector typically comprises a source of drive, such as a strong spring for moving a drive member, for example a rod, which acts on the plunger of the syringe. The prefilled syringe has a needle or cannula that is permanently attached to a first end of a syringe barrel or reservoir that is sealed at the opposing end by the plunger.

For safety and hygiene reasons it is desirable that the needle does not protrude from a housing of the autoinjector with the exception of the time when the needle is used for injection of a medicament. Therefore, either the autoinjector moves the needle out of the housing for the injection and back into the housing after injection or the autoinjector provides a needle cover or needle guard which may be moved to unsheathe the needle for an injection and which may be moved back in a needle covering position after the injection.

The majority of autoinjectors are configured as single use devices which incorporate both the syringe and the drive mechanism in the same housing. Such devices are usually disposable for ease of use.

In order to reduce costs and complexity it is desirable to design injection devices with a small number of parts and components. From an ecological aspects it is desirable to reduce the waste and to reuse devices as long as possible. In order to account for both ecological responsibility and medical and safety standards autoinjectors are developed as two-part or semi-disposable or semi-reusable devices. Such autoinjectors comprise a part which includes the syringe or reservoir with the drug and which can be separated and disposed of separately from the other part, for example, from a drive unit including a drive mechanism of the autoinjector.

EP 3930793 A1 discloses an autoinjector with a housing which can be separated after use of the autoinjector. A first housing part houses the syringe, a plunger rod and parts of the drive mechanism and a second housing part comprises an electronic unit including a sensor, a PCB and a power supply. After use the second housing part with the electronics can be disposed of separately from the fist housing part allowing for a correct waste separation.

EP 3019220 B1 discloses an autoinjector comprising a drive unit and a control unit which are assembled after a syringe has been inserted into the housing. When the autoinjector is pressed against an injection site, a needle shroud is moved proximally causing a plunger rod to rotate relative to the housing from an initial position to a release position. After the rotation the plunger rod is free to move axially in dispensing direction under the force of a drive spring.

WO 2010/000559 A1 discloses an autoinjector comprising a tubular elongated housing. Inside a hollow plunger rod is arranged wherein a spiral spring is arranged within the plunger rod. A guide for the springe is arranged inside the spring. The autoinjector comprises a rotatable manual knob at a proximal end. Rotation of the knob causes a rotation displacement of a container holder and this in turn causes mixing of the medicament.

GB2560558A discloses an autoinjector with a housing and a trigger button inserted into a rearward portion of the housing to close the rearward end of the hosing. The button has a cup-like profile with side walls. The housing includes a drive mechanism comprising a plunger rod which is arranged to engage a bund inside the syringe. The plunger rod is driven in dispensing direction by a pair of concentric drive springs.

### DESCRIPTION OF THE INVENTION

It is an objective of the invention to provide an autoinjector with a simple design which is easy to assemble and which is optimized for waste separation after use.

This objective is achieved by an injection device or a method according to the independent claims. Preferred embodiments are evident from the dependent claims.

The invention relates to an injection device for dispensing a liquid drug from a prefilled syringe through an injection needle permanently attached to a syringe barrel. The injection device comprises a pre-assembled drive unit connected to a pre-assembled syringe unit. The pre-assembled syringe unit comprises
- a syringe holder adapted to hold the syringe such that the syringe cannot move relative to the syringe holder or relative to any other part of the injection device, the syringe holder defines a longitudinal axis;
- a needle cover movable along the longitudinal axis relative to the syringe holder between a needle covering position in which the needle cannot be accessed from outside and a retracted position in which the needle protrudes from the needle cover,

The pre-assembled drive unit comprises
- a plunger rod;
- a spring member adapted to bias the plunger rod in a dispensing direction and adapted to move the plunger rod in dispensing direction during injection;
- a supporting member adapted to support or guide the spring member or/and the plunger rod;
- a first and a second retaining element, wherein each of the first and second retaining element can be in a holding position in which the first or second retaining element holds the biased plunger rod in place relative to the supporting member and in a release position in which the first or second retaining element does not hold the biased plunger rod in place.

Prior to assembly of the injection device the pre-assembled drive unit and the pre-assembled syringe unit are separated from each other and the first retaining element is in its holding position. Therefore, the biased plunger rod is exclusively held in place by the first retaining element in the pre-assembled drive unit.

During connection of the pre-assembled syringe unit to the pre-assembled drive unit a movement of the needle cover (and syringe holder) relative to the supporting member causes the first retaining element to move out of its holding position, preferably by a deflection or release movement of the first retaining element.

When injection device is completely assembled the drive unit is connected to the syringe unit and the needle cover is operatively coupled (directly or indirectly via intermediate member) to the second retaining element wherein
- the first retaining element is in its released position and thus does not hold the plunger rod anymore and
- the second retaining element is in its holding position such that the biased plunger rod is exclusively held in place by the second retaining element when the needle cover is in its covering position.

Prior to assembly of the injection device the syringe unit and the drive unit are pre-assembled. That means the pre-assembled syringe unit and the pre-assembled drive unit can be stored, handled and transferred as separate units before a final assembly step. This facilitates the storage and production of the injection device.

The syringe holder or/and the needle cover preferably include a holding member holding the syringe holder and the needle cover together such that they cannot drop off and cannot be separated. The holding member may be, for example, a snap-fit element, a clip or a thread. The same applies for the pre-assembled drive unit. That means the plunger rod, the spring member, the supporting member and the first and second retaining element are held together by a holding member. The components of the syringe unit and the components of the drive unit can thus be handled and stored together.

Furthermore, the pre-assembled drive unit and the pre-assembled syringe unit enable a simple and quick final assembly of the injection device.

After use the syringe unit and the drive unit may be disconnected by a skilled person and preferably by a dedicated tool rather than by the user. The tool-wise separation of the syringe unit from the drive unit provides, for example, a separation of components of different materials and thus a correct waste separation after use.

Moreover, the pre-assembled syringe unit and the pre-assembled drive unit according to the invention enable to bias the plunger rod and to hold the biased plunger rod in place prior to assembly, during assembly and in the completely assembled injection device. Namely, the first retaining element holds the plunger rod in place prior to assembly. During assembly, the holding function is transferred from the first holding element to the second holding element such that in a final state the plunger rod is exclusively held in place by the second retaining element and not held any more by the first retaining element.

After assembly, the biased plunger rod can be held in place depending on a needle cover position (retraced or covering position) as the second retaining element is directly or indirectly coupled to the needle cover when the syringe unit is connected to the drive unit.

During assembly at least a part of the drive unit is inserted into the syringe unit. A relative movement between the needle cover and the supporting member transfers the function of holding the plunger rod in place from the first to the second retaining element. That means a handing over of the held and biased plunger rod is quasi automatically conducted as no user action or additional assembly step is required to bring the first retaining element into its release position and subsequently to bring the second retaining element into its holding position. That further facilitates the assembly process of the injection device.

The relative movement during assembly of the injection device moves the first retaining element from its holding position into its released position. That may be, for example, by a deflection or by a disengagement or release movement of the retaining element. By way of example, a cam, protrusion or release member on a counterpart may contact or engage the first retaining element during the relative movement to bring the first retaining element in its released position.

During the period when the first retaining element is released and brought out of its holding position or subsequently or straight after this release the second retaining element is brought into its holding position or kept in its holding position. That means the second retaining element may be in its holding position or it may be actively brought into its holding position such that it can hold the biased plunger rod in place. During this transition phase the spring member may expand a a very short distance, for example 1mm or less.

Additionally, the second retaining element is directly or indirectly coupled to the needle cover, for example, by a coupling sleeve or by a coupling element such as a coupling cam, hook, protrusion or snap fit element during connection of the syringe unit with the drive unit.

The first and second retaining element are adapted to hold the biased plunger rod in place such that it cannot move in dispensing direction. The first and second retaining element may be implemented, for example, in form of a clamp, holding or retaining arm, protrusion or form-fit element.

The supporting member is adapted to support and guide the plunger rod along the longitudinal axis, preferably it guides the plunger rod such that it can move exclusively along the longitudinal axis and prevents a rotational or radial movement of the plunger rod. The supporting member may include, for example, a rib, a groove. Instead, or additionally, the supporting member may support or guide the spring member such that a reliable compression and relaxation of the spring member is enabled.

When the pre-assembled drive unit is connected to the pre-assembled syringe unit an injection device is formed. That means the syringe unit or the drive unit alone are not considered to be an injection device. The injection device is made if the pre-assembled syringe unit is connected to the pre-assembled drive unit.

The injection device is preferably a disposable automatic injection device such as an autoinjector. Autoinjectors usually comprise a syringe and an automatic drive mechanism to move a piston inside the syringe upon actuation of the autoinjector. The syringe is preferably a prefilled syringe that has a needle or cannula permanently attached to a distal end of a syringe barrel or reservoir and that is sealed at the opposing end by a piston.

The spring member preferably provides a spring force. By way of example, the spring member may be a metallic solenoid or spiral spring, a flat spring or leaf spring or an elastomer element or an elastically deformable plastic member adapted to provide a spring force in a biased state.

The syringe holder is adapted to hold the syringe with the liquid drug in place. That means the syringe holds the syringe relative to a housing and relative to the drive unit in a fixated manner and in particular prevents any axial movement of the syringe relative to the housing and drive unit during dose dispensing.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the injection device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In a preferred embodiment, in the assembled state of the injection device the second retaining element is in its holding position when the needle cover is in the covering position and the second retaining element is in its release position when the needle cover is in the retracted position. The second retaining element is thus coupled to the needle cover or the position of the second retaining element is depending on the needle cover position.

That means the biased plunger rod is held in place when needle is covered and thus access to the needle is not enabled. When the user moves the needle cover from the covering position into the retracted position (for example by a push on skin activation) the plunger rod may be released and the spring member can release and thereby moving the plunger rod in dispensing direction to dispense the drug from the syringe.

The supporting member preferably further comprises
- A connecting element adapted to connect the pre-assembled drive unit to the pre-assembled syringe unit;
- A guiding element adapted to guide the plunger rod along the longitudinal during dispensing. Preferably the guiding element is adapted to guide the plunger rod such that the plunger rod cannot rotate relative to the supporting member.
- A support surface for supporting the spring member at a proximal end of the spring member.

The supporting member as a single part fulfils thus a plurality of functions, namely a connecting, a guiding as well as a supporting function. This provides for a simple design and facilitates the assembly process as the number of components is reduced. Furthermore, the first and second retaining element are preferably connected to or integrally formed in the supporting member. That again combines different functions in one single supporting member.

The connecting element to connect the drive unit to the syringe unit is preferably a deflectable snap-fit member or a recess adapted to accommodate a snap-fit member. That means the syringe unit provides the other of the snap-fit member or the recess. The connection by means of the snap-fit member and the recess is thus a form-fit connection. That enables a quick and reliable connection between the syringe unit and the drive unit. The snap-fit connection further enables a disconnection of the syringe unit from the drive unit after use, preferably by a dedicated tool. The syringe unit and the drive unit may thus be separated in order to be disposed of separately.

The guiding element may be, for example, a rib, a groove, a rail, a dovetail guide or a form-fit engagement. The guiding element ensures a reliable movement of the plunger rod in dispensing direction during injection. The support surface provides a proximal end support for the spring member.

Preferably, the supporting member includes a sleeve-shaped portion comprising a circumferential wall defining a bore, wherein the biased plunger rod is at least partly arranged inside the bore. This enables a space-saving design and a facilitates the assembly as the spring member and the plunger rod may be accommodated at least partially inside the bore. If the supporting member includes a guiding element for the plunger rod the guiding element may be position within the bore.

Furthermore, in a preferred embodiment the first and preferably also the second retaining element are arranged in the wall and preferably integrally formed in the supporting member wall. Thus, the manufacturing of the supporting member (preferably by injection moulding) is facilitated as the body of the supporting member and the retaining elements can be produced on one single production step.

Preferably, the first retaining element is an arm extending along the longitudinal axis with a retaining portion on a free end of the arm. The retaining arm is preferably deflectable in a radial direction to release the plunger rod from its biased position such that the plunger rod can move in dispensing direction. That means the first retaining arm can be moved between its holding position and its release position by a radial deflection. The retaining portion of the arm may be a knob, a cam or a hook adapted to engage a recess or counter element in the plunge rod.

In a preferred embodiment also the second retaining element is an arm extending along the longitudinal axis with a retaining portion on a free end. The first and second retaining element formed as arms provide a simple and reliable holding mechanism to hold the biased plunger rod in place.

In a preferred embodiment the plunger rod is hollow defining a cavity and wherein the spring member is arranged inside the cavity. Preferably, the supporting member further comprises a guiding pin extending along the longitudinal axis inside the spring member and inside the cavity of the hollow plunger rod to guide the spring member. That provides a simple and space-saving design. Alternatively, the plunger rod may be rod-shaped and the spring member, in particular a spiral spring is arranged outside and may envelop the rod-shaped plunger rod.

Preferably, the pre-assembled syringe unit further comprises a sleeve-shaped housing and wherein the needle cover is coaxially arranged inside the housing and the syringe holder is arranged inside the needle cover. Thus, the housing is the outermost member of the pre-assembled syringe unit. Accordingly, when the injection device is assembled the housing is the outermost member of the injection device.

A cross-section of the housing may be circular but may have alternatively an elliptic, polygonal or rectangular shape. The housing and the syringe holder are preferably coaxially arranged. Both parts are preferably directly connected to each other, for example, by a form-fit, snap-fit or a threaded connection preventing any relative movement between the syringe holder and the housing. As the housing is directly connected to the syringe holder there is no need for an intermediate member and thus the syringe unit can be assembled with a reduced number of parts.

The housing and/or the syringe holder may include a guiding member to guide a movement of the needle cover along the longitudinal axis. However, the needle cover is held such that it cannot fall off and cannot be removed from the assembled housing and syringe holder. The syringe holder and/or the hosing may include two stop elements restricting a movement of the needle cover in both directions to prevent a separation of the latter.

In a preferred embodiment the housing includes a release surface adapted to move the first retaining element out of its holding position and into its release position by the relative movement during connection of the pre-assembled syringe unit with the pre-assembled drive unit. The first retaining element may be deflected, rotated, screwed or linearly moved out of its holding position by the release surface. The release surface may be, for example, a ramp or a sloped surface.

The biased plunger rod is therefore released from the first retaining element. The plunger rod is subsequently held in place by the second retaining element of the supporting member. The second retaining element may be held in place and held in its holding position by a locking surface of the needle cover.

That means during assembly of the pre-assembled syringe unit to the pre-assembled drive unit the first retaining element is released and the biased plunger rod is released. The plunger rod is then held in place by the second retaining element. The second retaining element is prevented from being moved out of its holding position and thus locked in the holding position by the locking surface of the needle cover.

The drive unit may comprise a second spring member adapted to bias the needle cover into the covering position. The second spring member is preferably held in a biased state by a holding element of the supporting member and preferably arranged on an outside of the supporting member.

The biased second spring may act directly or indirectly onto needle cover to ensure that the needle cover is kept in its covering position before an injection and that an unintentional retraction of the needle cover is prevented. To uncover the needle the needle cover has to be moved from the covering position into the retracted position against a force of the second spring member.

The holding element may be, for example, a flexible arm, snap element or rim portion adapted to hold the second spring member in place in a biased state. The second spring member may be released from the holding element upon attachment of the drive unit to the syringe unit such that the second spring member can engage the needle cover.

In a preferred embodiment a movement and in particular an axial movement of the needle cover and in particular of the locking surface relative to the second retaining element enables the retaining element to move out of its holding position.

The movement of the needle cover may be caused by a needle cover retraction movement, e.g. when the user presses a distal end of the injection device against the injection site and thereby moving the needle cover from the covering position towards the retracted position (also named as push-on-skin activation). The needle cover preferably moves along the longitudinal axis and the second retaining element is preferably deflectable in a radial direction or a direction angled to the longitudinal direction.

The housing comprises a connecting portion located proximally to the syringe holder which connecting portion extends along the longitudinal axis at least a half, preferably a full length of the axial length of the syringe holder. The connecting portion is adapted to accommodate and to be connected to the drive unit to form the injection device.

As the connecting portion extends along the longitudinal axis the pre-assembled drive unit can be at least partially inserted into housing connecting portion. Preferably, the supporting member is enveloped almost along its full axial length. That means the connecting portion accommodates and envelops the supporting member and thus the drive unit when the drive unit is connected to the syringe unit. The drive unit is thus protected from environmental impacts without requiring an additional housing. Moreover, the assembly is facilitated as no additional outer housing is required.

The connecting portion comprises preferably a connecting element such as, for example, a snap arm, protrusion or recess to form a form-fit connection with the connecting element of a supporting member of the pre-assembled drive unit.

Preferably, the drive unit further comprises a lock sleeve movable relative to the supporting member and adapted to lock, in the assembled state, the second retaining element and preferably additionally the first retaining element in its holding position such that the second retaining element and preferably also the first retaining element cannot be brought into its release position when the injection device is assembled.

The lock sleeve thus ensures that the second retaining element (and preferably also the first retaining element) remain in the holding position and thus the biased plunger rod is not unintentionally released. The lock sleeve may be arranged on a sleeve-shaped portion of the supporting member and is preferably movable along the longitudinal axis to lock or unlock the first and second retaining element.

During connection of the syringe unit with the drive unit the lock sleeve is preferably moved in along the longitudinal axis by a member of the syringe unit thereby releasing the lock and moving the first retaining element out of its holding position.

Preferably, the lock sleeve is operatively coupled to the needle cover such that a movement of the needle cover is transferred to the lock sleeve and vice versa.

Before assembly, the lock sleeve is preferably in a first or initial locking position and the needle cover spring is arranged between (a distal flange of) the lock sleeve and the supporting member.

During connection of the pre-assembled syringe unit with the pre-assembled drive unit the lock sleeve is preferably moved into an unlocking position in which the first retaining element is enabled to move from its holding position into its release position.

The lock sleeve includes preferably a deflectable locking arm or a recess to engage a deflectable arm and the needle cover comprises the other of the arm or the recess. The arm may be configured to deflect during insertion of the supporting member into a connecting portion of a housing of the syringe unit such that the arm engages the recess and thereby axially coupling the needle cover of the pre-assembled syringe unit to the lock sleeve of the pre-assembled drive unit.

In a preferred embodiment, when the needle cover is moved in its retracted position the lock sleeve is moved in a proximal end position wherein a return movement of the lock sleeve back in distal direction is prevented by the first or second retaining element. In the proximal end position, the lock sleeve is preferably adapted to lock the needle cover in the covering position by a snap-fit connection arranged in a distal portion of the lock sleeve and in a proximal portion of the needle cover. This prevents a second retraction of the needle cover and thus provides for a reliable needle protection.

In a preferred embodiment the supporting member comprises an elastic element on its distal end adapted to bias the syringe inside the syringe holder in an assembled state of the injection device. The elastic element is preferably integrally formed in the supporting member such that the elastic element and the supporting member from a monolithic part.

That provides a simple and compact design. Furthermore, the supporting member and the elastic element can be produced in only one production step, in particular by injection moulding. The elastic element may be formed by windings of spiral-shaped element.

The supporting member, the first and second retaining element, the plunger rod and, if any, the lock sleeve of the pre-assembled drive unit are preferably made of the same material, preferably polyoxymethylene (POM). That enables to dispose of the supporting member after use together with the plunger rod in the same manner or in the same waste. Polyoxymethylene as material is optimal in view of friction, costs and availability.

Moreover, the syringe holder, the needle cover and, if any, the housing, of the pre-assembled syringe unit are preferably made of the same material. These components are preferably made of polypropylene (PP) as this material is optimal in view of costs and availability.

The invention further relates to a method for assembly an injection device for dispensing a liquid drug from a syringe. The method comprises the steps of
- Inserting a distal portion of a pre-assembled drive unit as described above into a proximal connecting portion of a housing of a pre-assembled syringe unit as described above;
- Releasing, by the insertion movement, the first retaining element from the plunger rod and engaging the plunger rod by the second retaining element;
- Connecting the drive unit supporting member to the syringe unit housing by the connecting
   element to establish a snap-fit connection between the supporting member and the housing.

The present invention further relates to a pre-assembled syringe unit connectable to a drive unit as described above to build an injection device for dispensing a liquid drug from a reservoir through an injection needle. The syringe unit comprises
- a housing defining a longitudinal axis;
- a syringe holder adapted to hold a syringe non-movable along the longitudinal axis relative to the housing;
- a needle cover movable along the longitudinal axis relative to the syringe holder between a needle covering position and a retracted position.

The syringe holder is directly connected to the housing, for example by a form-fit or snap-fit connection, such that the syringe holder cannot move relative to the housing. The needle cover is supported or held between the syringe holder and the housing such that the needle cover can move along the longitudinal axis between the covering position and the retracted position and cannot fall off or cannot separated from the syringe holder or from the housing.

As described in detail above the housing of the pre-assembled syringe unit preferably comprises on a proximal side of the syringe holder a connecting portion which extends along the longitudinal axis at least a half, preferably a full length of the axial length of the syringe holder and the connecting portion is adapted to accommodate the supporting member and can be connected to the drive unit.

As the needle cover is held by the syringe holder and/or the housing the needle cover cannot drop off and the pre-assembled syringe unit can be stored and handled as one single unit or component. This facilitates the handling and assembly.

Due to the pre-assembled syringe unit the injection device can be assembled by inserting a syringe into the syringe holder and by connecting the pre-assembled syringe unit with the pre-assembled drive unit. Hence, the two pre-assembled units enable a simple and quick final assembly of the injection device.

The invention further relates to a pre-assembled drive unit for an injection device for dispensing a liquid drug from a syringe through an injection needle, wherein the drive unit is connectable to a pre-assembled syringe unit and wherein the drive unit comprises
- a plunger rod movable along a longitudinal axis;
- a spring member for biasing the plunger rod in a dispensing direction,
   wherein the drive unit further includes a supporting member comprising
- a connecting element to connect the drive unit to the pre-assembled syringe unit;
- a guiding element adapted to guide the plunger rod along the longitudinal axis;
- a first movable retaining element for holding the biased plunger rod in place in a shipping state and prior to a connection of the drive unit with the syringe unit;
- a support surface adapted to support the spring member at a proximal end of the spring member, and wherein the supporting member further includes a sleeve-shaped portion comprising a circumferential wall defining a bore wherein the biased plunger rod is at least partially arranged inside the bore. Preferably, the first retaining element is arranged in the wall.

In a further embodiment of the invention the second retaining element is a deflectable arm comprising a first and a second cam oppositely arranged on a free end of the arm, wherein the first cam is adapted to engage the plunger rod and the second cam is adapted to engage a locking surface of the needle cover in an assembled state of the injection device.

In this embodiment the needle cover is preferably sleeve-shaped and includes a guiding track (next to the locking surface) adapted to guide the second cam, wherein the guiding track includes an enabling portion enabling a movement of the needle cover sleeve relative to the supporting member as the second cam can travel within the guiding track. Additionally, the guiding track includes a lock portion adapted to prevent movement of the needle cover sleeve relative to the supporting member as a movement of the second cam is blocked (preferably by a stop surface) in the lock portion. The guiding track is preferably U-shaped.

Preferably, the deflectable arm is adapted to deflect radially in a first plane through a centre longitudinal axis of the injection device and the arm is additionally adapted to deflect in a second plane offset the centre longitudinal axis and perpendicular to the first plane.

Furthermore, the supporting member may include a snap element adapted to hold a needle cover spring in a biased state between the snap element and a proximal end flange of the supporting member.

In a further embodiment the first retaining element is a cam or a recess adapted to accommodate the cam and wherein the cam or recess is arranged on the plunger rod and the other of the cam and the recess is arranged on the supporting member.

Preferably, also the second retaining element is a cam or a recess adapted to accommodate the cam and wherein the cam or recess is arranged on the plunger rod and the other of the cam and recess is arranged on the needle cover.

The plunger rod is preferably rotatable relative to the supporting member and wherein in a first rotational orientation of the plunger rod relative to the supporting member the first retaining element is in its holding position (and preferably the second retaining element is in its release position) and in a second rotational orientation of the plunger rod the first retaining element is in its release position (and preferably the second retaining element is in its holding position).

The plunger rod comprises preferably a sloped wall or an engagement element adapted to engage the sloped wall and the needle cover or the housing comprises the other of the sloped wall or engagement element and wherein when the plunger rod is moved in dispensing direction the engagement element slides along the sloped wall thereby causing the plunger rod to rotate relative to the supporting member to move between the first and second rotational orientation.

In a further embodiment the syringe unit comprises a needle cover lock member having a recess and a stop wall. The lock member is preferably rotatable relative to the needle cover (and relative to the supporting member) and wherein the lock member is rotationally coupled to the plunger rod. In the first rotational orientation of the plunger rod the recess of the lock member is rotationally aligned with a protrusion of the needle cover such that the needle cover is free to move between the covering position and the retracted position. In the second rotational orientation of the plunger rod the lock member stop wall is rotationally aligned with the protrusion such that the needle cover can move in its covering position but is being prevented to move back into its retracted position.

In an alternative embodiment the plunger rod is exclusively rotated during assembly of the injection device when the pre-assembled syringe unit is connected to the pre-assembled drive unit. In particular, the plunger rod is rotatable to release the first retaining element and to engage the second retaining element as described above. In this alternative embodiment, the plunger rod is held rotationally fixed relative to the supporting member during the injection process. To release the plunger rod for the injection the second retaining element is preferably moved out of its holding position (deflected, rotated, axially moved) such that the plunger rod is free to move axially in dispensing direction without rotation.

The present invention further relates to a needle cover lock for an injection device for dispensing a liquid drug from a syringe through an injection needle, the cover lock comprising
- a supporting member defining a longitudinal axis;
- a needle cover movable along the longitudinal axis relative to the supporting member between a needle covering position and a retracted position in which the needle protrudes from the needle cover,
- a lock member movable relative to the supporting member and being in contact or coupled (directly or indirectly) with the needle cover such that an axial movement of the needle cover is transferred to the lock member,
   wherein the lock member comprises a stop element adapted to selectively engage a counter element on the supporting member,
   wherein in a first rotational orientation of the lock member relative to the supporting member the stop element does not encounter the counter element such that the lock member is movable along the longitudinal axis,
   wherein in a second rotation orientation of the lock member the stop element encounters the counter element thereby preventing a movement of the lock member and the needle cover towards the retracted position,
   and wherein a movement of the needle cover from the retracted position towards its covering position (and a distal movement of lock member) causes the lock member to rotate from its first rotational orientation into its second rotational orientation.

This arrangement provides a simple and reliable lock of the needle cover in the covering position and thus an unintentional access to the injection needle can be prevented.

The lock member may be rotated between the first and second rotational orientation by a force provided by a spring. The spring may be, for example, a mechanical spiral spring or an elastomer element initially mounted to the supporting member.

Preferably, the lock member is sleeve-shaped and comprises on its inside a guide track or a guiding cam adapted to be guided in the guiding track and the supporting member comprises the other of the guiding cam and the guide track. Such a cam control with a cam and a guiding track provides a reliable control of the movements of the lock member. That means the lock sleeve can be axially guided to prevent rotation or the guiding track can be design such that the lock sleeve rotates at a specific axial position.

The guiding cam is preferably a deflectable arm. The stop member is preferably arranged inside the guide track and the counter element is arranged on the cam.

The lock member has preferably a first linear guide to axially guide the lock member in the first rotational orientation and a second linear guide to axially guide the lock member in the second rotational orientation. The linear guide may be the guide track or a separate guide element. The lock member may include additionally an angled guiding section causing the rotation from the first rotational orientation into the second rotational orientation when the lock member is moved distally. The guiding cam may be adapted to engage the angled guiding section to initiate the rotation of the lock member.

The stop element is preferably a sawtooth comprising a sloped surface adapted to let pass the deflectable arm on a first side and a stop surface adapted to encounter the counter element on a second side. The sawtooth-shaped stop member provides a reliable locking of the needle cover in the covering position.

Preferably, the supporting member comprises an elastic element on its distal end adapted to bias a syringe inside a syringe holder. The elastic element is preferably integrally formed in the supporting member as a monolithic part.

In a preferred embodiment the needle cover lock further comprises a needle cover spring adapted to bias the needle cover in its covering position (in distal direction). The supporting member may include a holding element to couple lock member and the supporting member such that the biased needle cover spring can be held in a biased state between the lock member and the supporting member prior to assembly of the injection device. That means the supporting member, the lock member, and the needle cover spring form a pre-assembled unit which can be stored and handled as one unit. Additionally, the needle cover spring may be used to provide a force to rotate the lock member if released as described above.

The invention relates further to the injection device comprising the above described needle cover lock. The injection device preferably comprises a pre-assembled syringe unit and a pre-assembled drive unit. The two unit can be assembled, stored and handled separate from each other. The two units may be connected to each other to form the injection device. The pre-assembled syringe preferably includes
- a housing defining a longitudinal axis,
- a syringe holder arranged inside the housing and
- the needle cover
   wherein the pre-assembled drive unit preferably includes
- a plunger rod,
- a spring member adapted bias the plunger rod in a dispensing direction,
- the lock member and
- the supporting member.

The housing may be sleeve-shaped and the needle cover is preferably coaxially arranged inside the housing and the syringe holder is arranged inside the needle cover.

Preferably, the housing comprises a connecting portion as described above located proximally to the syringe holder which connecting portion extends along the longitudinal axis at least a half, preferably a full length of the axial length of the syringe holder. The connecting portion is adapted to accommodate and to be connected to the drive unit.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a perspective view of the components of an autoinjector according to a first embodiment of the present invention;
- Fig.2: depicts a perspective view of a pre-assembled syringe unit and a pre-assembled drive unit of the first embodiment;
- Fig.3a, 3b: depicts a sectional view of a cap of the autoinjector of figure 1 and 2;
- Fig. 4: depicts a sectional view of the autoinjector during assembly of the syringe unit and the drive unit;
- Fig. 5a: depicts a sectional view of the autoinjector in an initial state;
- Fig. 5b: depicts a sectional view in plane 90° rotated compared to the view of figure 5a;
- Fig. 6: depicts a sectional view of the autoinjector with a cover sleeve in the retracted position;
- Fig. 7a: depicts a sectional view of the autoinjector just after dispensing;
- Fig. 7b: depicts a sectional view of an alternative version of the first embodiment of the autoinjector with a lock sleeve with proximally extending locking arms;
- Fig. 8: depicts a sectional view of the autoinjector after dispensing with the needle cover sleeve locked in the covering position;
- Fig. 9: depicts a perspective view of the components of an autoinjector according to a second embodiment of the present invention;
- Fig. 10: depicts a perspective view of the syringe unit and drive unit according to the second embodiment;
- Fig. 11, 12: depict the drive unit and the plunger rod of the second embodiment;
- Fig. 13, 14: depict the autoinjector of the second embodiment in an initial state;
- Fig. 15, 16: depict the autoinjector after dispensing;
- Fig. 17: depicts a perspective view of the components of an autoinjector according to a third embodiment;
- Fig. 18: depicts a perspective view of the syringe unit and drive unit of the third embodiment;
- Fig. 19, 20: depict a perspective view of the lock sleeve and the supporting member;
- Fig. 21, 22: depict a sectional view of the autoinjector of figure 18 in an initial state;
- Fig. 23: depicts a sectional view of the autoinjector after dispensing with the cover sleeve in the retracted position;
- Fig. 24: depicts a sectional view of the autoinjector after dispensing with the cover sleeve locked in the covering position;
- Fig. 25: depicts a version of the third embodiment with a plunger rod comprising saw teeth;
- Fig. 26: depicts a further version of the third embodiment with click arms sliding over the injection spring windings;
- Fig. 27: depicts a perspective view of the components of an autoinjector according to a third embodiment;
- Fig. 28: depicts a perspective view of the syringe unit and drive unit of the third embodiment;
- Fig. 29, 30: depict a perspective view of the plunger rod and the supporting member;
- Fig. 31: depicts a sectional view of the autoinjector during assembly;
- Fig. 32: depicts a sectional view of the autoinjector in an assembled state;
- Fig. 33: depicts a perspective view of the autoinjector during assembly;
- Fig. 34: depicts a sectional view of the autoinjector in an assembled state;
- Fig. 35: depicts a sectional view of the autoinjector after dispensing with the cover sleeve in the retracted position and
- Fig. 36: depicts a sectional view of the autoinjector after dispensing with the cover sleeve locked in the covering position.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

In the following the term "distal" refers to the side where an injection needle is located. That is the left-hand side in the figures. The term "proximal" refers to the opposite side which is furthest away from the needle which is the right-hand side in the figures.

### First embodiment

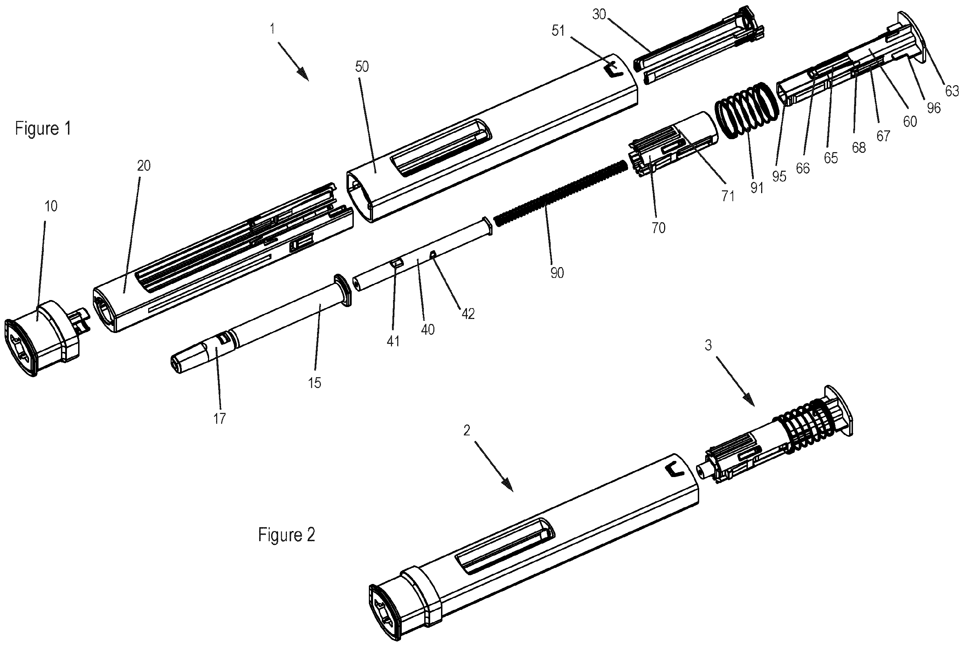

Figure 1 depicts a perspective exploded view of an injection device according to a first embodiment of the present invention in form of an autoinjector 1. The autoinjector 1 comprises a pre-assembled syringe unit 2 and a pre-assembled drive unit 3 (figure 2). During assembly a prefilled syringe 15 is inserted into a syringe holder 30 and the syringe unit 2 is attached and connected to the drive unit 3 to assemble the autoinjector 1 as described below. Figure 2 depicts a perspective view of the pre-assembled syringe unit 2 separated from the pre-assembled drive unit 3.

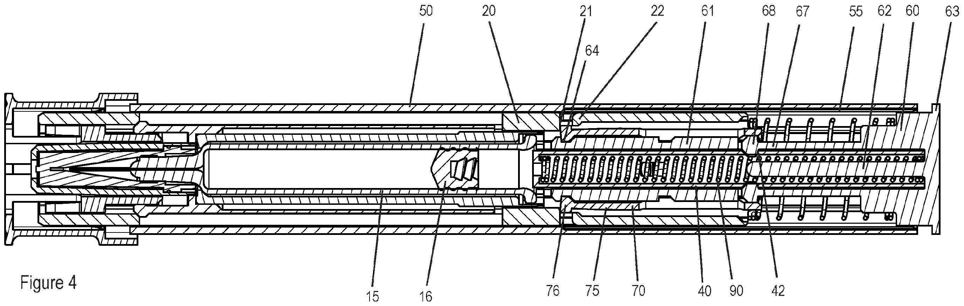

The pre-assembled syringe unit 2 as shown in figure 1 and figure 4 comprises a sleeve-shaped housing 50, a needle cover in form of an elongated sleeve 20, a cap 10 mounted on a distal end, the syringe holder 30 adapted to hold the syringe 15 such that it cannot move relative to the housing and the prefilled syringe (PFS) 15 containing a liquid drug and a piston 16 which is movable inside a syringe barrel. A rigid needle shield (RNS) 17 is initially mounted on the injection needle 18. The housing, the needle cover sleeve, the cap and the syringe holder are injection-molded plastic parts preferably made of polypropylene (PP).

The drive unit 2 (figure 1 and 4) comprises a supporting member 60, a hollow plunger rod 40, an injection spring 90 to move the plunger rod 40 in dispensing direction, a cover sleeve spring 91 adapted to bias the needle cover sleeve 20 in a covering position if the syringe unit 2 is connected to the drive unit 3 and a lock sleeve 70 arranged on an outside of a sleeve-shaped portion 61 of the supporting member 60. The supporting member, the plunger rod and the lock sleeve are injection-molded plastic parts and in particular made of polyoxymethylene (POM) or another technical plastic.

In the following the assembly of the autoinjector 1 is described. Subsequently, the function and the injection with the autoinjector is described in detail.

In order to assemble the pre-assembled syringe unit 2 the needle cover sleeve 20 is inserted into the housing 50. As the housing 50 has a rectangular cross section the needle cover sleeve 20 cannot rotate relative to the housing but is movable along the longitudinal axis. Then, the cap 10 is mounted on a distal end of the needle cover sleeve 20.

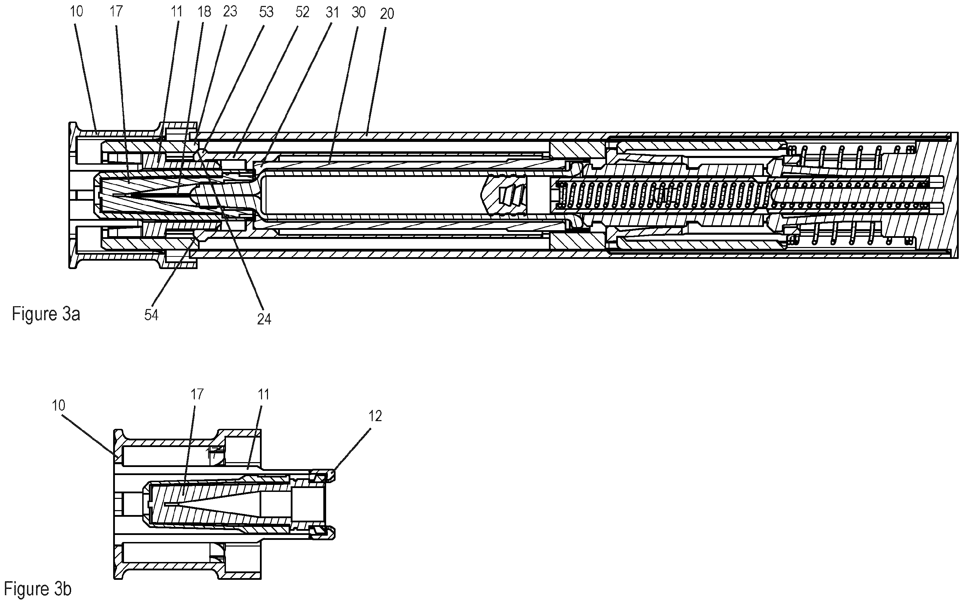

Figure 3a shows a sectional view of the autoinjector with the cap mounted onto the distal end of the needle cover sleeve 20. Figure 3b shows a section view through the cap 10 when it is separated from the autoinjector. The plane for the sectional view in figure 3b is 90° rotated compared to the plane for the sectional view of figure 3a.

As shown in figure 3a and 3b the cap 10 comprises inside a RNS remover sleeve 11 extending along the longitudinal axis and fixedly connected to an outer portion of the cap. The remover sleeve 11 includes a rim with hooks 12 (figure 3b) adapted to engage a proximal end of the RNS 17. If the cap 10 is mounted as shown in figure 3a the remover sleeve 11 is radially between the RNS 17 and securing arms 52 of the housing 50. The securing arms 52 have free ends comprising knob-shaped ends 53. The securing arms 52 are arranged radially between an inner side of the needle cover sleeve 20 and an outside surface of the remover sleeve 11. The needle cover sleeve 20 includes securing rips 23 extending along the longitudinal axis and including proximally faced sloped surfaces 24 that engage corresponding counter sloped surfaces 54 on the knob ends 53 of the securing arms 52. As the surfaces 24 engage the counter surfaces 54 and as the securing arms 52 are held in place by the remover sleeve 11 the needle cover sleeve 20 is prevented from being moved proximally.

As a next assembly step, the syringe holder 30 is inserted from a proximal side into the housing 50 but not yet moved into a distal end position. The housing 50 includes a connecting portion 55 (see figure 4) extending proximally of the inserted syringe holder and having a length of about the axial length of the syringe holder 30. This connecting portion 55 enables to accommodate the supporting member 60 when the syringe unit is connected to the drive unit.

After the above assembly step, the syringe unit 2 is in its pre-assembled state and all components of the syringe unit are fixated and cannot fall off and cannot be separated unless a (snap-fit) connection is released.

At the drug manufacturer site or at the assembly site the prefilled syringe 15 with its RNS 17 is inserted from the proximal side into the syringe holder 30 and deflects a distal rim 31 (figure 3a) of the syringe holder 30 to let pass the RNS 17. The rim 31 then deflects back into its original position and thus the rim 31 is located between the proximal end of the RNS 17 and a distal shoulder of the syringe barrel. The syringe holder 30 together with the syringe 12 is then moved into its final distal position within the housing 50 until the holder abuts a stop rib of the housing 50 as shown in figure 3a. In this final position the rim 31 is prevented from being deflected or moved radially outwards by the housing which encounters the rim 31 and holds the rim in place.

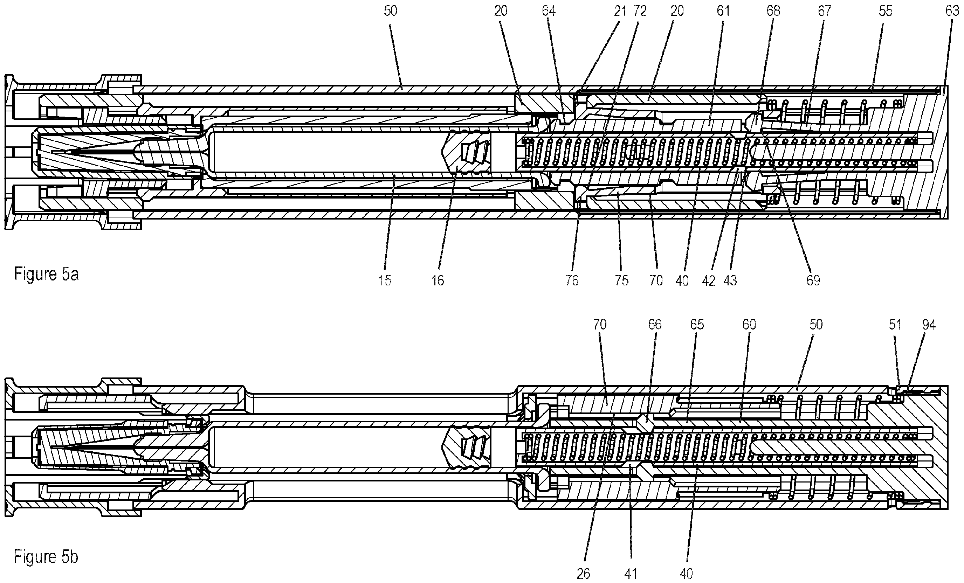

For the pre-assembly of the drive unit 3 the injection spring 90 in form of a spiral spring is inserted into a bore or opening of the sleeve-shaped portion 61 of the supporting member 60 such that the windings of the spring 90 are located radially between an inner guiding pin 62 and the inner wall of the sleeve-shaped portion 61 as shown in figure 4. Subsequently, the plunger rod 40 is inserted with its proximal end into the sleeve-shaped portion 61 until knobs 66 at the end of distal retaining arms 65 snap into distal recesses 41 in the plunger rod (see figure 5b) and additionally knobs 68 of proximal retaining arms 67 snap into proximal recesses 42 in a side wall of the plunger rod (shown in figure 4 and in released state in figure 5a). The two distal retaining arms 65 are oppositely arranged to each other and the two proximal retaining arms 67 are oppositely arranged to each other. Moreover, the two pairs of retaining arms 65, 67 are 90° rotated relative to each other. A perspective view of the supporting member 60 with the distal and proximal retaining arms 65, 67 with their respective knobs 67,68 can be seen in figure 1. After this assembly step the injection spring 90 in compressed state is exclusively held in place by the proximal retaining arms 67.

Then, the cover sleeve spring 91 is mounted to the sleeve shaped portion 61 and shifted in proximal direction until the proximal end of the cover sleeve spring 91 abuts a support surface 96 on a supporting rib shown in figure 1. As a final assembly step, the lock sleeve 70 is mounted onto the sleeve-shaped portion 61 and moved towards a proximal end wall 63 until cam ends 76 of deflectable locking arms 75 of the lock sleeve 70 snap into distal recesses 64 in the supporting member 60 (as shown in figure 4) such that the lock sleeve 70 is held onto the supporting member by a snap-fit connection.

The drive unit 3 is then in its pre-assembled state as shown in figure 2. That means all components of the pre-assembled drive unit 3 are fixated and cannot fall off and thus cannot be separated unless a snap-fit connection is released. That is, the cover sleeve spring 91 is compressed and held in place by a ledge 71 of the lock sleeve and the latter is held onto the supporting member by its locking arms 75. The injection spring 90 is compressed and biases the plunger rod 40 in distal dispensing direction. The plunger rod 40 is held in place by the proximal retaining arms 67 and the arms 67 in turn are held in their holding position by the lock sleeve 70 as an inner surface of the lock sleeve abuts the knobs 68 and thus prevents the proximal retaining arms 67 from being deflected radially outwards.

As a last assembly step for assembly the autoinjector the pre-assembled syringe unit 2 with the inserted syringe is connected to the pre-assembled drive unit 3. For that purpose, the sleeve-shaped portion 61 is inserted into the connecting portion 55 of housing 50 from a proximal side. Figure 4 depicts a state where the drive unit 3 is partly inserted but not yet completely connected to the syringe unit 2.

As a proximal end of the needle cover sleeve 20 is located proximally to the ledge 71 the cover sleeve spring 91 directly encounters the needle cover sleeve 20. That means the cover sleeve spring 91 is held in its compressed state exclusively by the needle cover sleeve 20 and thus biases the cover sleeve in its covering position when the drive unit is connected to the syringe unit.

As shown in figure 4 during assembly the locking arms 75 encounter a ledge 21 inside the needle cover sleeve 20. When the drive unit 3 is further moved distally into the housing 50 the lock sleeve 70 is stopped and does not move any further as cam ends 76 engage with the ledges 21. That means the supporting member 60 together with the plunger rod 40 and the injection spring are moved relative to the lock sleeve 70 and hence relative to the housing 50. That in turn causes the locking arms 75 to deflect radially outwards as the arms include sloped surfaces 72 (figure 5a) on their free ends and as the cam ends 76 are pushed against the ledges 21. The cam ends 76 are thereby locked to the cover sleeve 20 as each cam end 76 move in gap between the ledge 21 and a distal snap element 22 of the needle cover 20.

Furthermore, as the knobs 68 of the proximal retaining arms 67 include a proximally oriented sloped surface 69 engaging a distally oriented sloped surface 43 in proximal recess 42 of the biased plunger rod 40 (see figure 5a) the retaining arms 67 are forced to deflected radially outwards as the injection spring moves the plunger rod 40 a short distance and hence the sloped surfaces 69, 43 slide along each other pressing the retaining arms 67 radially outwards. This in turn means the plunger rod 40 is released from proximal retaining arms 67. The plunger rod 40 is then exclusively held in place by the distal retaining arms 65 engaging the distal recesses 41 in the plunger rod (see figure 5b).

Figure 5a and 5b show a fully assembled autoinjector 1 where the drive unit 3 is completely inserted into the syringe unit 2 and connected thereto. As shown the supporting member 60 is completely inserted into the connecting portion 55 such that a proximal end wall 63 closes the proximal opening of the housing 50. A distal end of the supporting member 60 abuts a flange portion of the syringe 15 and thereby fixates the syringe 15 inside the autoinjector. As shown in figure 5a the proximal retaining arms 67 are deflected radially outwards.

Figure 5b shows the same state of the autoinjector 1 as figure 5a but the cut for the sectional view is in a plane 90° rotated compared to the plane of the sectional view of figure 5a. As shown in figure 5b a proximal end portion of the supporting member 60 is connected to the proximal end of the housing by a snap element 51 in the housing 50 engaging a protrusion 94 of the supporting member 60. The supporting member 60 is thus fixated to the housing 50 and cannot move relative thereto.

In figure 5b it can be seen that the knobs 66 of the distal retaining arms 65 are within the distal recesses 41 of the plunger rod 40 and thus secure and hold the plunger rod in place. The distal retaining arms 65 are prevented from being deflected outwards by an inner surface 26 of the needle cover sleeve 20.

In order to prepare the autoinjector for an injection the user has to remove the cap 10. As the remover sleeve 11 of the cap engages the RNS 17 with its hooks 12 (see figure 3b) the RNS is removed together with the cap 10 if the use pulls off the cap from the autoinjector.

When the cap 10 is removed from the needle cover sleeve 20 the securing arms 52 of the housing 50 are no longer prevented from being deflected radially inwards. That means if a force acts on the needle cover sleeve in proximal direction the needle cover sleeves 20 can move proximally thereby deflecting the knob ends 53 and the securing arms 52 can deflect radially inwards as the sloped surfaces 24, 54 slide along each other.

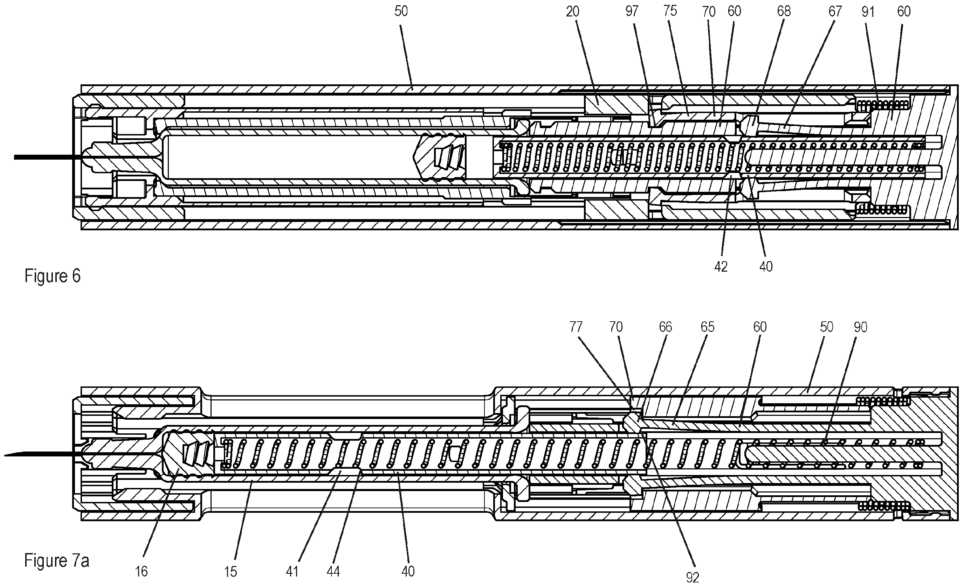

To start the injection the user presses a distal end of the needle cover sleeve 20 onto the injection site and thereby pushes the needle cover sleeve 20 into the housing against the spring force of the cover sleeve spring 91. The cover sleeve 20 moves thus from its covering position into its retracted position (see figure 6). This movement triggers several functions.

Firstly, the proximal movement of the needle cover sleeve 20 also moves the lock sleeve 70 in a proximal end position as the lock sleeve is axially connected to the needle cover sleeve, shown in figure 6 or 7a. That causes the distal retaining arms 65 to get out of engagement with the inner surface of the lock sleeve 70. As the knobs 66 of arms 65 have proximally oriented sloped release surfaces 92 sliding against corresponding distally oriented release sloped surfaces 44 of the distal recess 41 of the plunger rod (figure 7a) the knobs 66 are forced radially outwards into a distal recess 77 in the lock sleeve 70 as shown in figure 7a. Therefore, the distal retaining arms 65 deflect radially outwards and thus release the plunger rod 40. That means the compressed injection spring 90 can release and thus move the plunger rod 40 in dispensing direction to move the piston 16 inside the syringe barrel to dispense the drug from the syringe 15. The plunger rod is axially guided by the sleeve-shaped portion 61 which has a rectangular cross-section with rounded corners 95 (see figure 1) corresponding to a plunger rod proximal end flange. The plunger rod 40 is thus prevented from being rotated.

Secondly, the radially deflected distal retaining arms 65 further hold and lock the lock sleeve 70 in the proximal end position such that the lock sleeve and cannot move again in distal direction.

Thirdly, as shown in figure 6 as the lock sleeve 70 is no longer pushed distally against the ledge 21 the locking arms 75 can deflect back radially inwards into a proximal recess 97 in the supporting member meaning that the axial connection between the needle cover sleeve 20 and the lock sleeve 70 is disengaged.

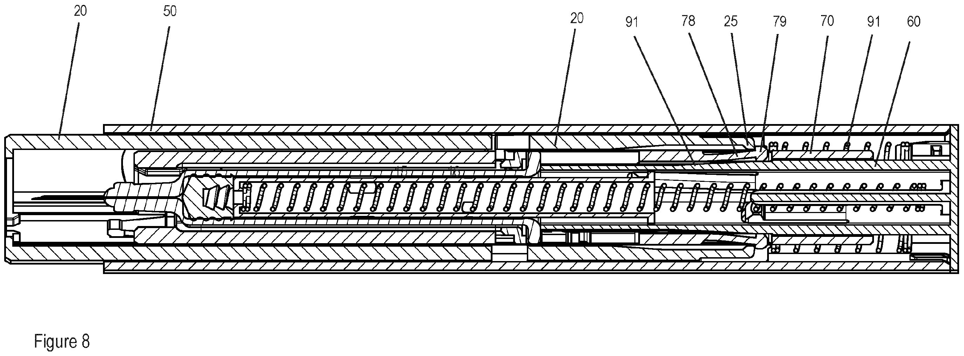

Fourthly, the proximal holding arms 78 with holding cams 79 of the lock sleeve 70 can deflected radially outwards means of a ramp 91 (figure 8) on an outside of the sleeve-shaped portion 61. As shown in figure 8 and as described below the holding cams 79 can engage the needle cover sleeve 20 to lock the cover sleeve 20.

Once the injection is completed the user removes the autoinjector 1 from the injection site. Due to the cover sleeve spring 91 the needle cover sleeve 20 is moved from the retracted position back into its distal covering position. This state of the autoinjector 1 is shown in figure 8. Shortly before the needle cover sleeve 20 reaches its final covering position proximal snap elements 25 of the needle cover sleeve 20 pass the lock sleeve holding cams 79. As the snap elements include an angled surface on a distal side but a stop surface on a proximal side the needle cover sleeve 20 can move distally but is prevented from being moved back in proximal direction once it has passed the deflected holding cams 79. The needle cover sleeve 20 is thus locked in its covering position (needle cover lock).

After use the autoinjector can be disassembled and the syringe unit plastic parts made of PP can be discarded of separately from the drive unit plastic parts made of POM.

Alternatively, the drive unit and preferably also the syringe unit without the syringe can be reused. In this case at a manufacturer site or reprocessing site a skilled person carries out a reset procedure to prepare the autoinjector for reissue to consumers.

Before disassembly the autoinjector may logged either by means of an electronic tag such as a NFC tag or by a serial number. If the lifetime is not yet exceeded the autoinjector is forwarded for resetting.

In a first reset step a specific disassembly tool is inserted next to the housing snap elements 51 to release the snap-fit connection between the housing 50 and the supporting member 60. Thus, the syringe unit 2 and drive unit 3 can be separated. The emptied syringe 15 is then removed from the syringe unit and disposed of.

In a next step, the lock sleeve 70 is disassembled from the supporting element 60 and the plunger rod 40 is shifted from a distal end position back into a proximal end position and thereby the injection spring 90 is biased again. The lock sleeve 70 is then mounted to the supporting element again and the plunger rod 40 is held in place by the proximal retaining arms 67 as described above.

In a further step, the injection spring force may be tested by releasing the plunger rod 40 to determine whether the injection spring 90 fulfills the requirements for a reissue. If the spring force does not reach a predefined threshold or if any malfunction of the drive unit 3 is detected the drive unit 3 is discarded and replaced by a new drive unit.

In a further step, the syringe unit 2 and the drive unit 3 are subject to an industrial cleaning or sterilization. Further, a new prefilled syringe 15 is inserted into the syringe holder 30. The syringe unit and the drive unit can then be connected again as described above to assemble the autoinjector for reissue.

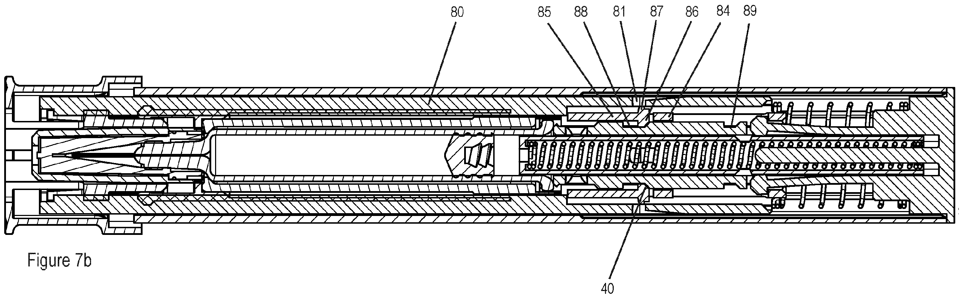

Figure 7b depicts a further version of the first embodiment with a different lock sleeve 84. In contrast to the lock sleeve described above the lock sleeve 84 shown in figure 7b has proximally extending locking arms 85 each having a cam end 86 on a proximal end. The needle cover sleeve 80 has a slightly modified recess 81 compared to the needle cover sleeve 20 described above.

Figure 7b shows the state of the autoinjector when the drive unit is connected to the syringe unit and the plunger rod is held in place by distal retaining arms. However, in the state shown in figure 7b the cam ends 86 of the locking arms 85 have not yet inserted into recess 81 in the cover sleeve 80 to axially couple the lock sleeve 84 to the cover sleeve. The distal recess 88 of the lock sleeve 84 comprises a sloped proximal end wall and the cam ends 86 comprise a corresponding sloped wall causing the locking arms to deflect radially outwards. Each cam end 86 include additionally a protrusion 87 adapted to engage into the recess 81 the facilitate the coupling to the needle cover sleeve 80.

The lock sleeve 84 further includes a second proximal recess 89 to accommodate the cam ends 86 after dispensing when the lock sleeve is in its distal end position as described above. All other functions are the same as described above with respect to the first version of the first embodiment.

### Second embodiment

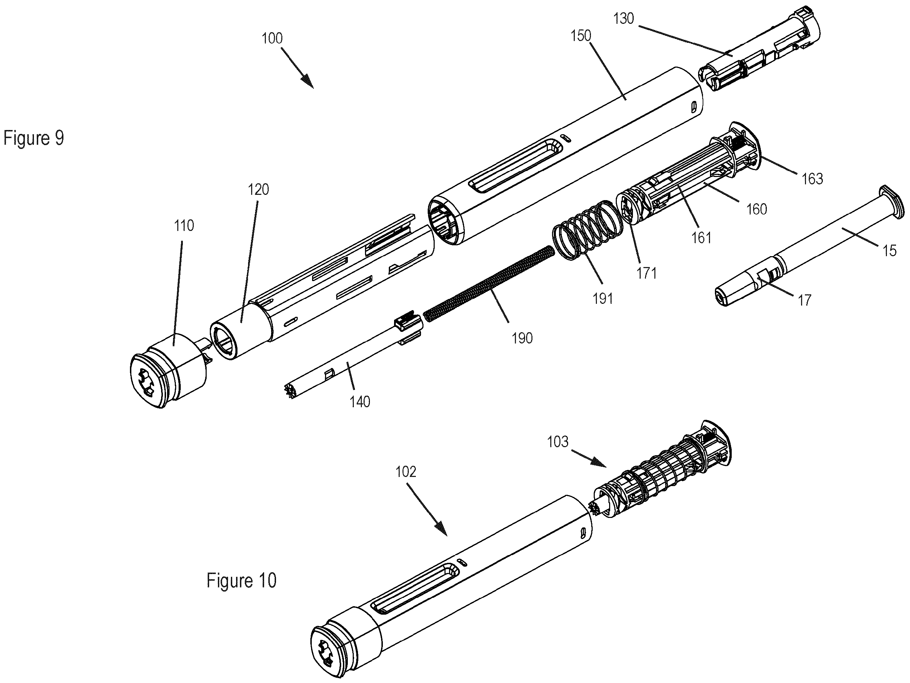

Figure 9 depicts a perspective exploded view of an injection device according to a second embodiment of the present invention in form of an autoinjector 100. The autoinjector 100 comprises a pre-assembled syringe unit 102 and a pre-assembled drive unit 103 (see figure 10). During assembly a prefilled syringe 15 is inserted into a syringe holder 130 and the syringe unit 102 is attached and connected to the drive unit 103 to assemble the autoinjector 100. Figure 10 depicts a perspective view of the pre-assembled syringe unit 102 separated from the pre-assembled drive unit 103 according to the second embodiment.

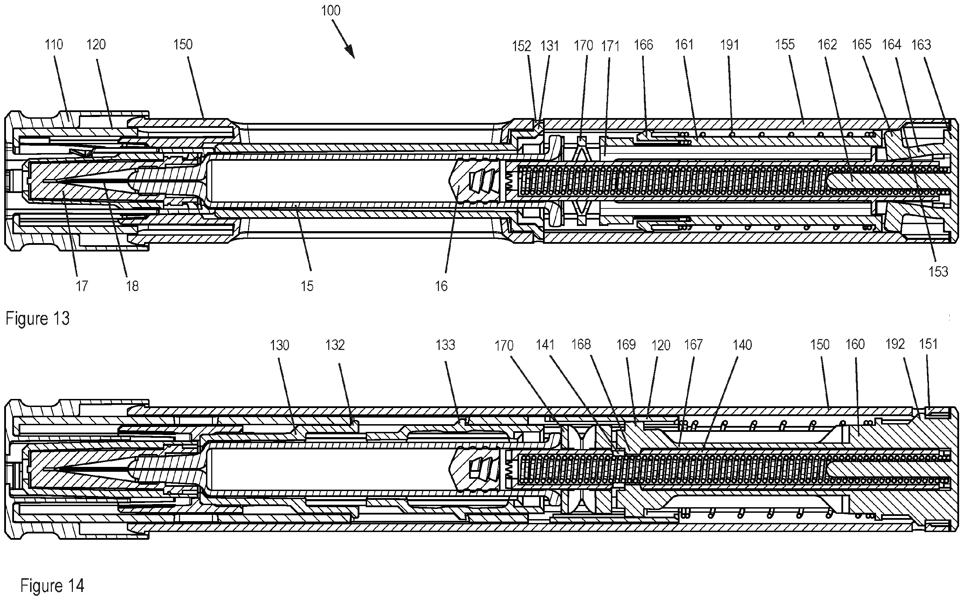

The syringe unit 102 as best shown in figure 9 and 13 comprises a sleeve-shaped housing 150, a needle cover sleeve 120, a cap 110, the syringe holder 130 adapted to hold the syringe 15 such that it cannot move relative to the housing and the prefilled syringe (PFS) 15 containing a liquid drug and a piston 16. A rigid needle shield (RNS) 17 is initially mounted on the injection needle 18. The housing 150, the needle cover sleeve 120, the cap 110 and the syringe holder 130 are injection-molded plastic parts and all made of polypropylene (PP).

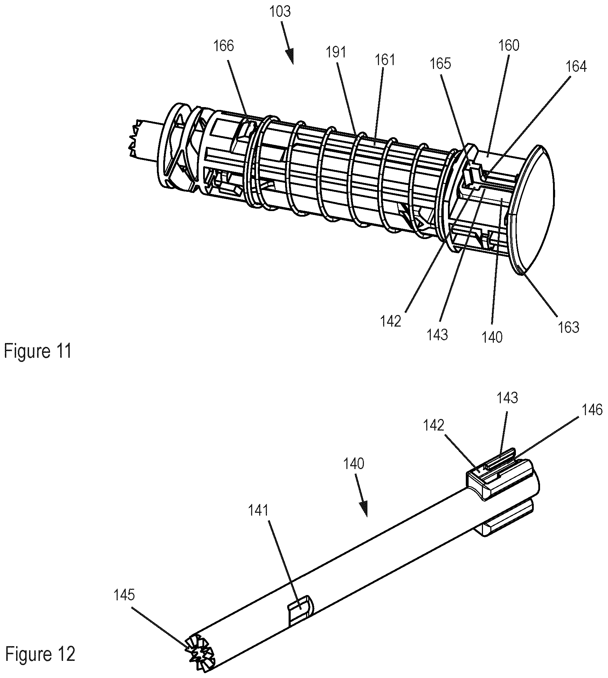

The drive unit 102 as shown in figure 9 and 11 comprises a supporting member 160, a hollow plunger rod 140, an injection spring 190 to move the plunger rod 140 in dispensing direction and a cover sleeve spring 191 adapted to bias the needle cover sleeve 120 in a covering position if the syringe unit 102 is connected to the drive unit 103. The supporting member 160 and the plunger rod 140 are injection-molded plastic parts and in particular made of polyoxymethylene (POM).

In order to assembly the syringe unit 102 the needle cover sleeve 120 is inserted into the housing 150 from a proximal side. The housing 150 has a rectangular cross section which prevents the needle cover sleeve 120 to rotate relative to the housing but the cover sleeve is movable along the longitudinal axis relative to the housing 150. Then, the cap 110 is mounted on a distal end of the needle cover sleeve 120. As a next assembly step, the syringe holder 130 is inserted from a proximal side into the housing 150 but it is not yet moved into a final distal position.

After the above assembly step, the syringe unit 102 is in its pre-assembled state and all components of the syringe unit are fixated and cannot fall off and cannot be separated unless a snap-fit connection is disengaged.

At the drug manufacturer site or at the assembly site the prefilled syringe 15 with its RNS 17 is inserted from the proximal side into the syringe holder 130. The syringe holder 130 together with the syringe 112 is then moved into its final distal position within the housing 150 until a distal flange 131 of the holder 130 snaps into a recess 152 in the housing 150 to establish a snap-fit connection, see figure 13. Furthermore, the syringe holder 130 includes in a middle portion and in a proximal portion two oppositely arranged noses 132, 133 that snap into a recess in the housing 150 to axially fix the syringe holder 130 as shown in figure 14.

Figure 11 shows a perspective view of the pre-assembled drive unit 103 and figure 12 depicts a perspective view of the plunger rod 140 alone. For the pre-assembly of the drive unit 103 the injection spring 190 in form of a spiral spring is inserted into a bore or opening 171 of a sleeve-shaped portion 161 of the supporting member 160 (figure 9 or 13) such that the windings of the spring 190 are located radially between an inner guiding pin 162 and the inner wall of the sleeve-shaped portion 161 (see figure 13). Subsequently, the hollow plunger rod 140 is inserted with its proximal end into the opening 171 until the plunger rod snaps into the supporting member. That is, the plunger rod 140 includes two oppositely arranged connecting portions 146 shown in figure 12. Each connection portion comprises two axially oriented holding ribs 143 and a recess 142 arranged on a distal end of the ribs 143 as best shown in figure 12. The supporting member 160 includes in a proximal portion two oppositely arranged deflectable L-shaped snap elements 164 (see figure 11) adapted to snap into the recess 142. The L-shaped snap elements 164 include a radially outwards protruding cam 165.

As shown in figure 11 the snap element 164 is positioned in the recess 142 distally to the holding ribs 143 and held in place by the holding ribs 143. As the injection spring 190 is compressed the plunger rod 140 is biased in distal direction and held in place by the snap elements 164 such that the plunger 140 rod cannot move relative to the supporting member 160. As shown in figure 12 the plunger rod 140 comprises on its distal end arrowhead-shaped protrusions 145 extending in distal direction and adapted to engage and enter in the syringe piston made of rubber.

As a next assembly step, the cover sleeve spring 191 is mounted to envelop the sleeve-shaped portion 161. The cover sleeve spring 191 is compressed and held in place by a radially protruding snap element 166 located in the sleeve-shaped portion 161, see figure 11.

The drive unit 103 as shown in figure 10 and 11 is then in its pre-assembled state. All components of the drive unit 103 are fixated and cannot fall off and thus cannot be separated unless a snap-fit connection is disengaged.

To assembly the autoinjector 100 the pre-assembled syringe unit 102 with the inserted syringe is connected to the pre-assembled drive unit 103. That is, the sleeve-shaped portion 161 is inserted into a connecting portion 155 of housing 150 from a proximal side as shown in figure 13.

Figure 13 depicts a sectional view of the completely assembled autoinjector 100 according to the second embodiment. In the assembled state the drive unit 103 is completely inserted and connected to the syringe unit 102. During insertion the cover sleeve spring 191 is released from the snap element 166 and encounters a proximal end of the needle cover sleeve 120. Therefore, the cover sleeve spring 191 biases the cover sleeve 120 in distal direction in a covering position.

Moreover, as shown in figure 13 during insertion the L-shaped snap elements 164 are deflected radially inwards. That is because the radial cams 165 of the snap elements 164 encounter sloped contact surfaces 153 inside the proximal housing forcing the cams 165 and thus the L-shaped snap elements 164 inwards. The deflection of the snap elements 164 in turn releases the snap-fit to the plunger rod 140 and thus the spring-biased plunger rod moves a short distance in distal direction until radially inwards extending protrusions 168 of two oppositely arranged retaining arms 167 engage proximal ends of oppositely arranged recesses 141 in the plunger rod 140.

The retaining arms 167 are shown in figure 14 which depicts the same state of the autoinjector 100 as figure 13 but the plane of the sectional view is 90° rotated compared to the plane of the sectional view of figure 13. Each radial protrusion 168 comprises further a radially outward extending guiding cam 169 which abuts an inner surface of the needle cover sleeve 120. The retaining arms 167 are thus prevented from being deflected radially outwards and thus the plunger rod 140 is held in place and locked by the retaining arms 167.

Furthermore, the supporting member 160 comprises on its distal end a spring element 170 which is monolithic with the sleeve-shaped portion 161 and thus integrally formed in the sleeve-shaped portion. When the drive unit 103 is connected to the syringe unit 102 a distal end of the spring element 170 encounters a proximal end of the syringe 15 and thus biases the syringe 15 in distal direction against a distal stop surface inside the syringe holder 130.

A proximal end portion of the supporting member 160 is connected to the proximal end of the housing 150 by a snap element 151 in the housing 150 (figure 14) engaging a nose 192 of the supporting member. The supporting member 160 is thus fixated to the housing 150 and cannot move relative thereto.

To start the injection the user removes the cap 110 and presses a distal end of the needle cover sleeve 120 onto the injection site. Thereby the needle cover sleeve 120 is pushed into the housing 150 against the spring force of the cover sleeve spring 191. The cover sleeve 120 is thus moved from its covering position into its retracted position (push-on-skin).

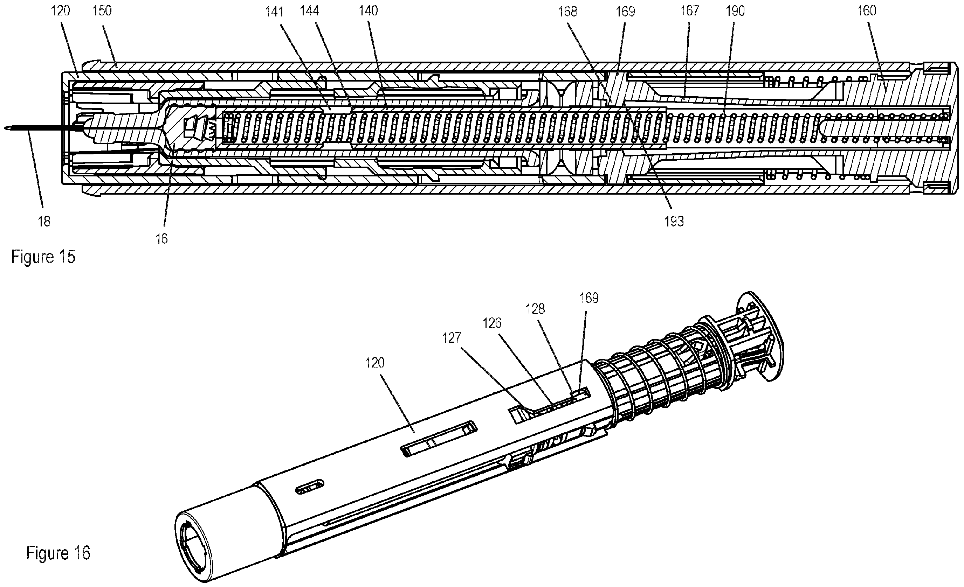

Figure 15 depicts a state when the cover sleeve 120 is in the retracted position and the autoinjector has completely injected the liquid drug.

When the needle cover sleeve 120 is in the retracted proximal end position each guiding cam 169 of the retaining arms 167 gets axially aligned with one of the two oppositely arranged U-shaped grooves 126 in the needle cover sleeve 120. This grooves 126 are depicted in figure 16 which shows a perspective view of the needle cover sleeve and the drive unit. Other components and in particular the housing of the autoinjector is not depicted in figure 16.

As it can be seen in figure 15 the two oppositely arranged recess 141 in the plunger rod have each a sloped proximal end surface 144 and as the protrusions 168 have corresponding sloped surfaces 193 the protrusions 168 are forced to move radially outwards and hence the retaining arms 167 deflect radially outwards. Thereby the guiding cams 169 enter the grooves 126 in distal area within the groove as can be seen in figure 16.

Due to the radially outwards deflected retaining arms 167 the plunger rod is released and thus the injection spring 190 can release and moves the plunger rod 140 in distal direction. The piston 16 inside the syringe barrel is then moved until a distal end position as shown in figure 15. Upon contact of the plunger rod distal end with the piston 16 the arrowheads 145 of the plunger rod tip enter the rubber of the piston thereby damping the impact of the plunger rod to the piston to avoid damage of the syringe barrel.

When the user removes the autoinjector 100 the needle cover sleeve 120 is moved from the retracted position back into its distal covering position due to the spring force of the cover sleeve spring 191.

The guiding cams 169 inside the U-shaped grooves 126 can move out of the distal area as a proximal side of the cam 169 as well as corresponding angled wall 127 in the groove 126 as shown in figure 16 are sloped with respect to the longitudinal axis. Therefore, as the guiding cam 169 can travel within the groove 126 the needle cover sleeve 120 can move distally back in its covering position. Due to the U-shape of the grooves 126 the guiding cams 169 and the retaining arms 167 are deflected laterally during the retraction movement. That means the retaining arms 167 are moved out of a plane running through the device center longitudinal axis and are deflected in a plane parallel and offset to the central longitudinal axis.

When the needle cover sleeve 120 has reached its covering position again and thus the distal end position the guiding cams 169 are moved laterally into a proximal locked position and encounter a stop wall 128 which is in alignment with the central longitudinal axis. This state is shown in figure 16. If a force acts on the needle cover sleeve 120 in proximal direction (towards the retracted position), e. g. when the device is pushed again onto a surface the needle cover sleeve 120 cannot move any more as the guiding cams 169 encounter the stop walls 128 and cannot exit the locked proximal position inside the grooves 126. This is because the stop walls 128 prevent any deflection or lateral movement of the guiding cams 169 (needle cover lock).

Similar as described with respect to the first embodiment the autoinjector according to the second embodiment can be disassembled and the parts made of the same material can be discarded of together.

Alternatively, and as described above the autoinjector may be reused after a reset procedure. Namely, the snap-fit connection between the supporting member 160 and the housing 150 can be released to separate the pre-assembled syringe unit 102 from the pre-assembled drive unit 103. The plunger rod 140 can be pushed back into a proximal end position to compress the injection spring 190. The plunger rod 140 can then be held in place by the L-shaped snap elements 164 as described above. Subsequently, a new syringe can be inserted in the syringe holder and the syringe unit can be reconnected to the drive unit.

### Third embodiment

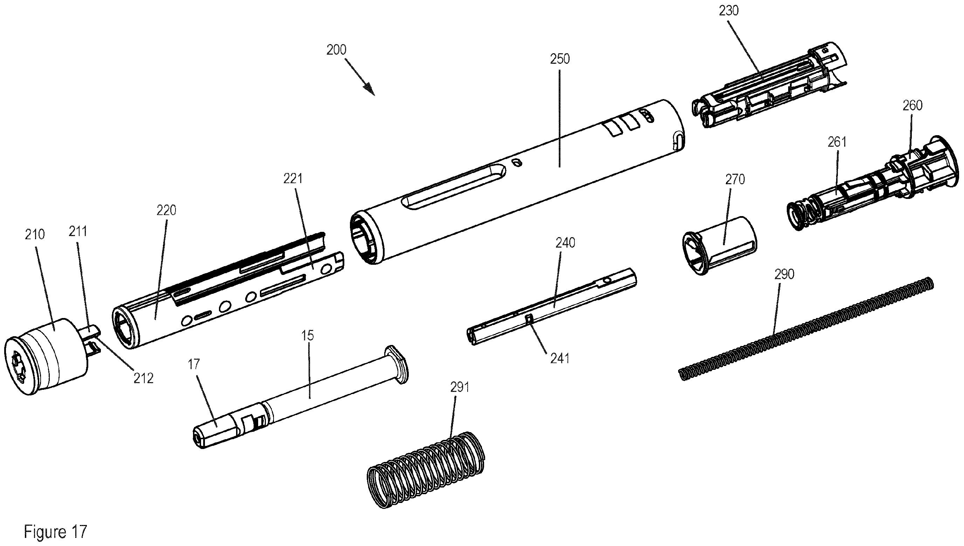

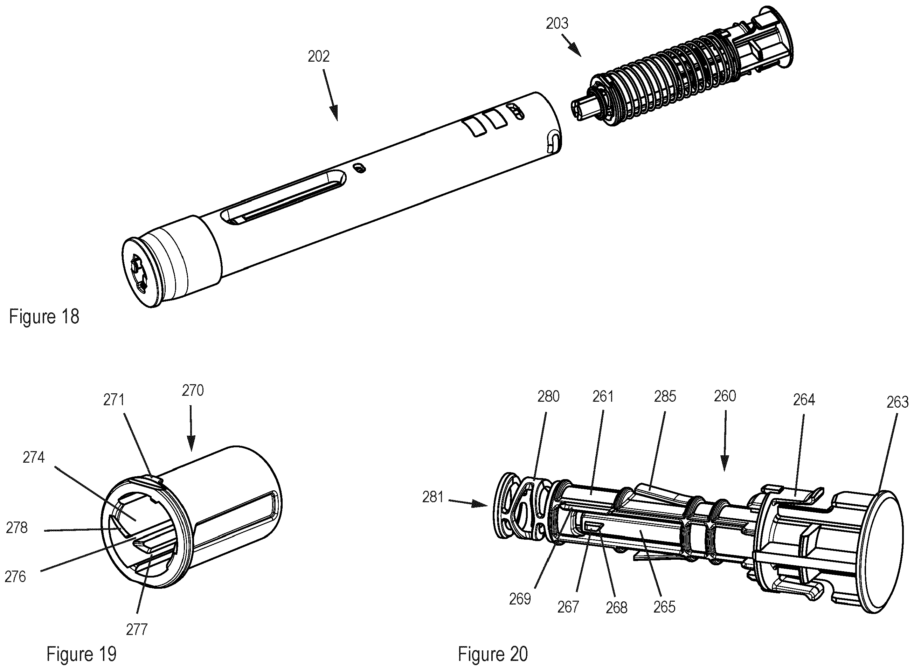

Figure 17 depicts a perspective exploded view of an injection device according to a third embodiment of the present invention in form of an autoinjector 200. The autoinjector 200 comprises a pre-assembled syringe unit 202 and a pre-assembled drive unit 203. Figure 18 depicts a perspective view of the pre-assembled syringe unit 202 separated from the pre-assembled drive unit 203.

The syringe unit 202 comprises a sleeve-shaped housing 250, a needle cover sleeve 220 in, a cap 210, a syringe holder 230 adapted to hold a syringe such that it cannot move relative to the housing and the prefilled syringe (PFS) 15 containing a liquid drug and a piston 16 which is movable inside a syringe barrel. A rigid needle shield (RNS) 17 is initially mounted on an injection needle 18. The housing, the needle cover sleeve, the cap and the syringe holder are injection-molded plastic parts preferably made of polypropylene (PP).

The drive unit 202 comprises a supporting member 260, a hollow plunger rod 240, an injection spring 290, a cover sleeve spring 291 and a lock sleeve 270 arranged on an outside of a sleeve-shaped portion 261 of the supporting member 260. The supporting member, the plunger rod and the lock sleeve are injection-molded plastic parts and in particular made of polyoxymethylene (POM).

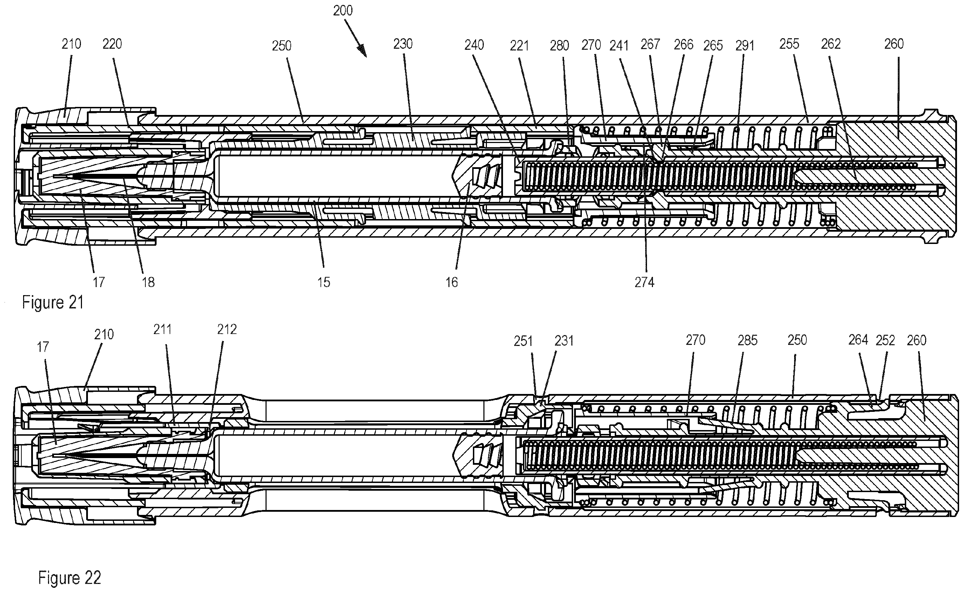

For the assembly of the syringe unit 202 the needle cover sleeve 220 is inserted into the housing 250. The housing 250 comprises on its inside longitudinal guiding rails (not shown) to guide the needle cover sleeve and to prevent rotation. Then, the cap 210 is mounted on a distal end of the needle cover sleeve 220. The cap includes two oppositely arranged and longitudinally extending tongues 211 with hooks 212 at their ends which can engage a proximal end of the RNS 17. The syringe holder 230 is then inserted from a proximal side into the housing 250. The syringe holder includes a two oppositely arranged cams 231 which snaps into a recess 251 in the housing (see figure 22) to form a snap-fit connection and thus the syringe holder 230 cannot move relative to the housing 250. The syringe unit 203 is then in its pre-assembled state and all components of the syringe unit are fixated and cannot be separated.

Figure 20 depicts a perspective view of the supporting member 260 alone. For the pre-assembly of the drive unit 203 the injection spring 290 in form of a spiral spring is inserted into a bore or opening 281 (figure 20) of the sleeve-shaped portion 261 of the supporting member 260 such that the windings of the spring 290 are located radially between an inner guiding pin 262 and the inner wall of the sleeve-shaped portion 261, shown in figure 21. Subsequently, the plunger rod 240 is inserted with its proximal end into the sleeve-shaped portion 261 until radially inwards extending protrusions 266 of retaining arms 265 snap into recesses 241 in the plunger rod (figure 21). The two retaining arms 265 are oppositely arranged to each other. Subsequently, the cover sleeve spring 291 is mounted onto the sleeve-shaped portion 261 and moved towards a proximal end wall 263.

Figure 19 depicts a perspective view of the lock sleeve 270 alone. The lock sleeve 270 is mounted onto the sleeve-shaped portion 261. Details of the lock sleeve 270 are described below.

The drive unit 3 is then in its pre-assembled state. The plunger rod 240 is held in place by the retaining arms 265 which in turn are held in a holding position by the lock sleeve 270 as the inner surface of the lock sleeve abuts a radially outwards protruding cam 267 of the retaining arms 265 and thus prevents the retaining arms 265 from being deflected. The lock sleeve 270 includes a spring retaining nose 271 on a distal end to hold the biased cover sleeve spring in place.

As shown in figure 19 the lock sleeve 270 comprises a first and a second axial guide in form of a first axial groove 276 and second axial groove 277 which are spaced apart from each other in a circumferential direction. The lock sleeve comprises in total two oppositely arranged first grooves 276 and two second grooves 277. The first and second axial grooves 276, 277 extend along the longitudinal axis and are arranged on an inside of the lock sleeve. Furthermore, an angled guide section 278 is arranged on an inner side of the lock sleeve 270. Moreover, the lock sleeve 270 comprises an engagement section with a locking surface 274 which protrudes radially inwards. The locking surface 274 is adapted to be in contact with cams 267 and holds the retaining arms 265 in their holding position.

The supporting member 260 comprises on an outer surface of the sleeve-shaped portion 261 an axial rib 269 adapted to be inserted into the axials grooves 276, 277. The two axial grooves define a first and a second rotational position of the lock sleeve 270 relative to the supporting member 260 (and the housing). That means the axial rib 269 can either be in the first or in the second axial groove 276, 277 and thus the lock sleeve 270 is in its first or second rotational position.

The supporting member 260 comprises the radially outwards protruding cams 267 on the free ends of the retaining arms 265. The cams 267 are adapted to be inserted into the first axial grooves 276 as described in detail below. As best shown in figure 20 each cam 267 has on its proximal side a wall 268 angled with respect to the longitudinal axis and adapted to cooperate with the angled guide section 278 of the lock sleeve 270.

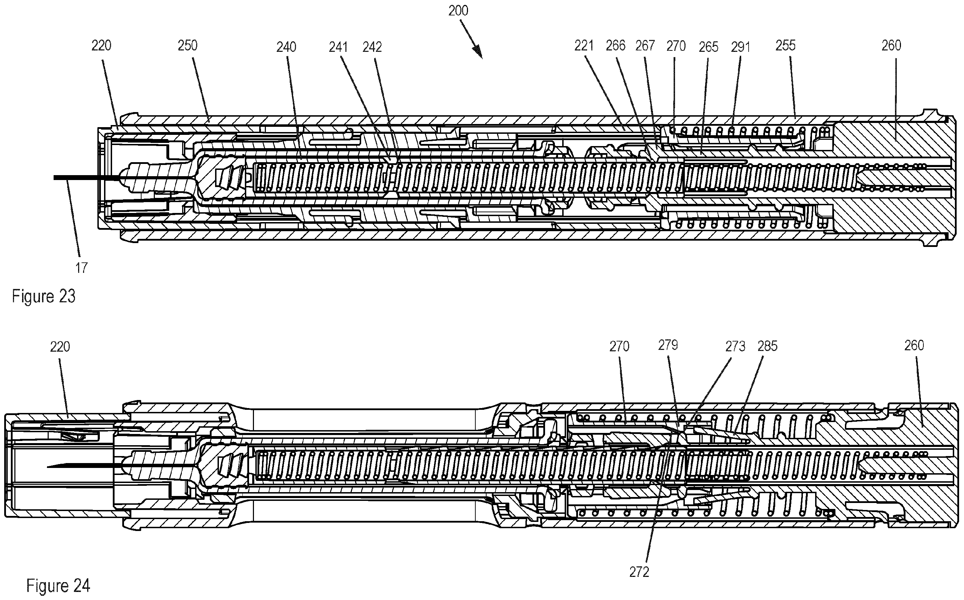

The two axial grooves 276, 277 are formed by two adjacent walls or portions that protrude radially inwards in the lock sleeve. That means in a section of the grooves the lock sleeve as a smaller inner diameter. In a section spaced apart from the grooves the lock sleeve as a larger inner diameter. This section with the larger inner diameter is adapted to accommodate locking arms 285 of the support member 260 which are best shown in figure 20. Moreover, the section with the larger inner diameter comprises two oppositely arranged saw teeth 279 protruding radially inwards, shown in figure 24. On a distal side each saw teeth 279 has a sloped surfaces 272 and on a proximal side the saw teeth comprises a stop surface 273 adapted to engage a free end of the locking arm 285. However, the two locking arms 285 are only engageable with the sawteeth 279 when the lock sleeve 270 is in its second rotational orientation as described further below.

As a last assembly step for assembly the autoinjector the pre-assembled syringe unit 202 with an inserted syringe 15 is connected to the pre-assembled drive unit 203. For that purpose, the sleeve-shaped portion 261 is inserted into a connecting portion 255 of the housing 250 from a proximal side.

Figures 21 and 22 depict the autoinjector in the assembled state where the drive unit 203 is completely inserted into the syringe unit housing 250 and connected thereto. Figure 22 depicts the same state as figure 21 but the plane of the sectional view is 90° rotated compared to the plane of the sectional view of figure 21.

The supporting member 260 comprises on its distal end a spring element 280 which is monolithic with the sleeve-shaped portion 261 and thus integrally formed in the sleeve-shaped portion. When the drive unit 203 is connected to the syringe unit 202 a distal end of the spring element 280 encounters a proximal end of the syringe 15 and thus biases the syringe 15 in distal direction against a distal stop surface inside the syringe holder 230.

As it can be seen in figure 22 a proximal end portion of the supporting member 260 is connected to the proximal end of the housing 250 by a snap arm 264 which engages a recess 252 in the housing such that the drive unit 203 is fixated to the housing 250 and cannot move relative thereto.

In figure 21 it can be seen that the cams 267 of the retaining arms 265 are held in place by the locking surface 274 of the lock sleeve 270 which prevents the retaining arms 265 from being deflected. The protrusions 266 are thus held in the recesses 241 (figure 1 and 21) and therefore the biased plunger rod 240 is held in place.

To prepare the autoinjector 200 for an injection the user has to remove the cap 210. The RNS 17 is removed together with the cap 10 as the tongues 211 with the hooks 212 engage the RNS (figure 17 and figure 22).

When the needle cover sleeve 220 is pressed onto the injection site the needle cover sleeve 220 is pushed into the housing against the spring force of the cover sleeve spring 291. The cover sleeve 220 moves thus from its covering position into its retracted position.

The proximal movement of the needle cover sleeve 220 also moves the lock sleeve 270 in a proximal end position as two extensions 221 of the needle cover sleeve encounter the lock sleeve 270, see figure 23. That means the locking surface 274 is shifted away from the cams 267 and the cams can get out of contact with the surface such that the retaining arms 265 are free to deflect radially outwards (not shown in figure 23). As the proximal end of the plunger rod recess 241 has a sloped surface 242 (figure 23) and as the protrusions 266 have corresponding proximal sloped surfaces the surfaces slide along each forcing the retaining arms 265 to deflect radially outwards due to the spring force of the compressed injection spring 290. That in turn means the plunger rod 240 is released and is moved in dispensing direction by the injection spring 290 until the piston 16 reaches a distal end within the syringe barrel. This state is shown in figure 23. Although not shown in figure 23 the protrusion 266 are not in engagement with the plunger rod anymore and remain on an outside surface of the plunger rod once the plunger rod is released.

Shortly before the lock sleeve 270 reaches its proximal end position the axial rib 269 of the supporting member 260 leaves the first axial groove 276 due to the proximally moved lock sleeve 270 and hence the lock sleeve is no longer axially guided and can be rotated relative to the supporting member.

When the injection is completed, the user removes the autoinjector 200 from the injection site. Due to the cover sleeve spring 291 the needle cover sleeve 220 is moved from its retracted position back towards its distal covering position. That means the lock sleeve 270 is no longer held in its proximal position and as the axial rib 269 is out of engagement with first axial groove 276 the lock sleeve is free to rotate.

As the retaining arms 265 are deflected radially outwards the angled walls 268 (shown in figure 20) of the cams 267 engages the angled guiding sections 278 (figure 19) of the lock sleeve. Due to the force of the compressed cover sleeve spring 291 the angled section 277 is pressed onto the angled wall 268 which forces the lock sleeve 270 to rotate relative to the supporting member 260 (and thus relative to the housing) from its first rotational position unit the axial rib 269 hits a stop rib next to second axial groove 277 and hence the axial rib 269 is rotationally aligned with second axial groove 277. Coincidently, the cam 267 is rotationally aligned with the first axial groove 276. The lock sleeve is now in its second rotational position. As the cover sleeve spring 291 pushes the lock sleeve 270 in distal direction the axial rib 269 enters the second axial groove 277 and the cam 267 enters the first axial groove 276 thereby rotationally locking the lock sleeve 270 in its second rotational position.

As shown in figure 24 in the second rotational position the saw teeth 279 are rotationally aligned with the locking arms 285 of the supporting member 260. That means when the lock sleeve 270 is further moved distally the free ends of the locking arms 285 pass the sloped surface 272 of the saw teeth 279 and snap into the space proximal to the saw teeth stop surface 273. This state is shown in figure 24 which is a sectional view in a plane 90° rotated to the plane of the sectional view of figure 23. That means the locking arms 285 cannot pass the stop surface 273 and hence the lock sleeve 270 is prevented from being moved back in distal direction. This in turn means the needle cover sleeve 220 cannot be moved towards its retracted position again and hence is locked in the covering position (needle cover lock).

Also, the autoinjector according to the third embodiment can be disassembled and the parts made of the same material can be discarded of together. Alternatively, as described above with respect to the first embodiment the autoinjector may be reused after a reset procedure.

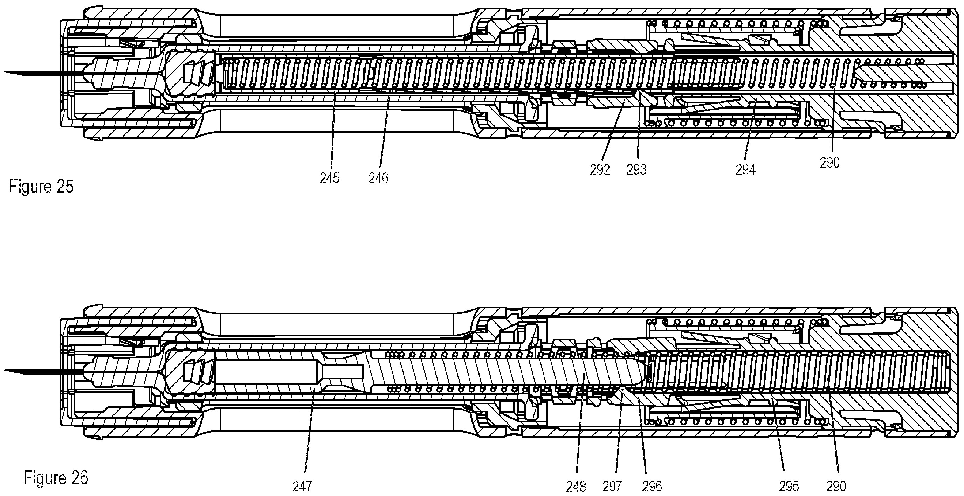

Figure 25 depicts a further version of the third embodiment. In contrast the version of the third embodiment described above the supporting member 294 of the autoinjector shown in figure 25 comprises additionally two oppositely arranged deflectable arms 292 each having a radially inwards extending nose 293. Furthermore, the plunger rod 245 comprises two oppositely arranged flattened portions comprising sawteeth 246 arranged next to each other in longitudinal direction. When the plunger rod 245 is released, and the injection spring 290 moves the plunger rod 245 in dispensing direction the noses 293 of the arms 292 glides over the saw teeth 246 thereby providing an audible and tactile feedback to the user during injection.

Figure 26 depicts a further version of the third embodiment. In contrast to the autoinjector 200 of the third embodiment described in above the version shown in figure 26 comprises a supporting member 295 without a guiding pin. Instead, a guiding pin 248 is integrally formed in the plunger rod 247 as shown in figure 26. That means the plunger rod 247 does not have a hollow shape which envelops the injection spring 290. The supporting member 295 comprises two oppositely arranged deflectable arms 296 each having a nose 297 on their free end adapted to engage windings of the injection spring 290.

When the plunger rod 247 is released the noses 297 of the arms 296 slide over the windings of the coil of the injection spring 290 and thus providing an audible and tactile feedback to the user during injection.

### Fourth embodiment

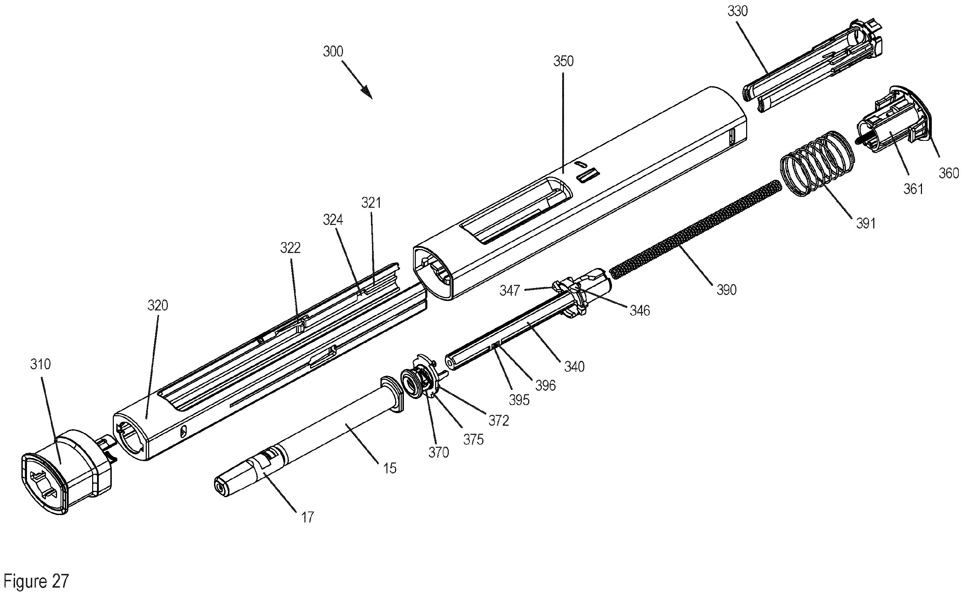

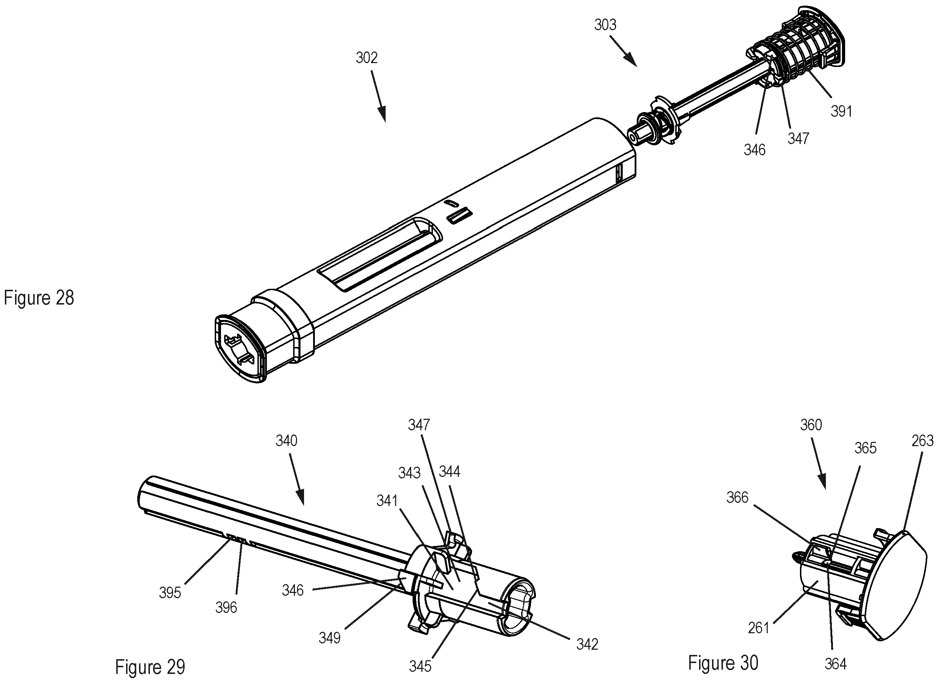

Figure 27 depicts a perspective exploded view of an injection device according to a fourth embodiment of the present invention in form of an autoinjector 300. The autoinjector 300 comprises a pre-assembled syringe unit 302 and a pre-assembled drive unit 303 as shown in figure 28.

The pre-assembled syringe unit 302 comprises a sleeve-shaped housing 350, a needle cover sleeve 320, a cap 310, a syringe holder 330 and the prefilled syringe (PFS) 15, see figure 27. A rigid needle shield (RNS) 17 is initially mounted on the injection needle 18. The housing, the needle cover sleeve, the cap and the syringe holder are injection-molded plastic parts preferably made of polypropylene (PP).

The pre-assembled drive unit 302 comprises a supporting member 360, a hollow plunger rod 340, an injection spring 390 to move the plunger rod 340 in dispensing direction, a cover sleeve spring 391 adapted to bias the needle cover sleeve 320 and a lock member 370 coupled to the plunger rod 340. The supporting member, the plunger rod and the lock member are injection-molded plastic parts and in particular made of polyoxymethylene (POM).

For the assembly of the pre-assembled syringe unit 302 the needle cover sleeve 320 is inserted into the housing 350. As the housing 350 has flattened sides and has thus has a non-circular cross section the needle cover sleeve 320 cannot rotate relative to the housing but is movable along the longitudinal axis. The cap 310 is mounted on a distal end of the needle cover sleeve 320.

As a next assembly step, the syringe holder 330 is inserted from a proximal side into the housing 350 but not yet moved into a distal end position. After the above assembly step, the syringe unit 302 is in its pre-assembled state and all components of the syringe unit are fixated and cannot fall off and cannot be separated unless a snap-fit connection is released.

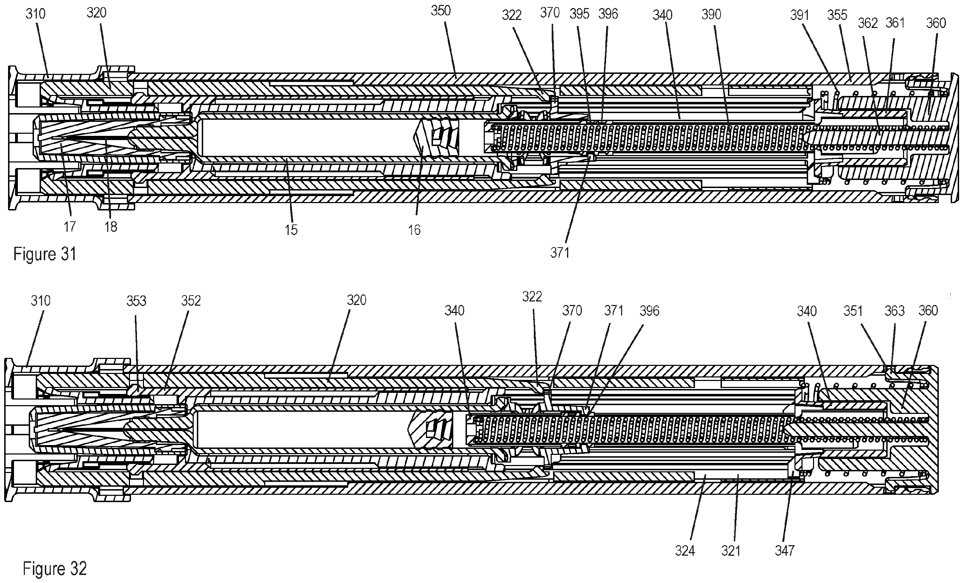

At the drug manufacturer site or at the assembly site the prefilled syringe 15 with its RNS 17 is inserted from the proximal side into the syringe holder 330. The syringe holder 330 together with the syringe 12 is then moved into its final distal position within the housing 350 as shown in figure 31.

For the pre-assembly of the drive unit 303 the injection spring 390 in form of a spiral spring is axially inserted into a bore or opening of the sleeve-shaped portion 361 of the supporting member 360 such that the windings of the spring 390 are located radially between an inner guiding pin 362 and the inner wall of the sleeve-shaped portion 361 as shown in figure 31. The cover sleeve spring 391 is mounted onto the sleeve-shaped portion 361 and then the plunger rod 340 is axially inserted into a distal opening of the sleeve-shaped portion.

Figure 29 shows a perspective view of the plunger rod 340 alone and figure 30 depicts a perspective view of the supporting member 360 alone. The plunger rod 340 includes in a proximal portion two oppositely arranged guiding tracks 341. Each guiding track 341 includes an axial groove section 342 and a radial extending section 343. The radial section 343 includes a distally oriented walls having a sloped lock surface 344 and an adjacent sloped release surface 345.

As shown in figure 30 the supporting member 360 includes on its sleeve-shaped portion 361 two oppositely arranged and radially inwards protruding cams 366 each having a sloped lock surface 364 on one side and a sloped release surface 365 on the other side. The two surfaces 364, 365 of each cam 366 are proximally oriented and arranged adjacent to each other such that each cam 366 is arrowhead shaped.

During assembly the cams 366 are inserted into the axial groove sections 342 and then the plunger rod 340 is rotated relative to the supporting member 360 such that the cams 366 enter the radial sections 343. As the plunger rod 340 is biased by the injection spring 390 in distal direction the sloped lock surface 344 of the plunger rod and the sloped lock surface 364 of the supporting member are pressed against each other. As the lock sloped surface 344 of the plunger rod is oriented away from a guide track inlet the plunger rod 340 is reliably held in place and coupled to the supporting member. That means during the assembly the plunger rod 340 is connected to the supporting member 360 by an axial and a subsequent rotational movement and thus the plunger rod 340 is connected to the supporting member 360 by a bayonet connection.

As a final step the lock member 370 is mounted to the plunger rod 340 from a distal side and shifted onto the rod unit two oppositely arranged clamping arms 371 of the lock member 370 snap into a first recess 395 in the plunger rod, see figure 31. The plunger rod includes a first distal recess 395 and a second proximal recess 396 which are also shown in figure 29. The lock member 370 is thus fixated relative to the plunger rod 340 and cannot rotate and not axially moved relative to the plunger rod.

The drive unit 303 is then in its pre-assembled state as shown in figure 28. All components of the pre-assembled drive unit 303 are fixated and cannot fall off and thus cannot be separated unless a snap-fit connection is released. That is, the cover sleeve spring 391 is compressed and held in place by two oppositely arranged first guiding cams 346 and two oppositely arranged second guiding cams 347 of the plunger rod 340. The latter is held on the supporting member by the above-described bayonet connection.

The pre-assembled syringe unit 302 with the inserted syringe 15 is then connected to the pre-assembled drive unit 303. The plunger rod 340 with the attached lock member 370 and the sleeve-shaped portion 361 of the supporting member is inserted into a connecting portion 355 (figure 31) of the housing 350 from a proximal side.

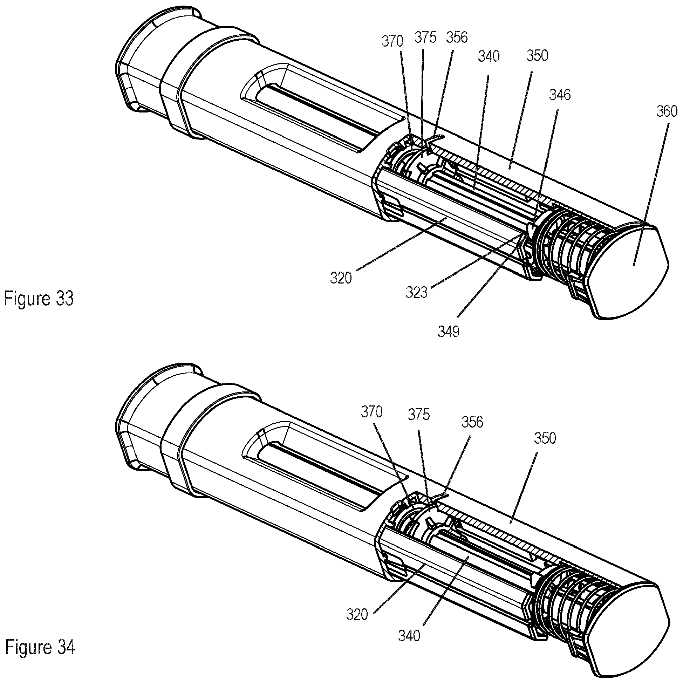

Figure 31 and figure 33 depict a state in that the drive unit 303 is partly inserted but not yet completely connected to the syringe unit 302. Figure 31 shows a sectional view of the autoinjector and figure 33 depicts a corresponding perspective view. In figure 33 one half a proximal housing section is not drawn to allow an inspection of the inner parts of the autoinjector.

As best shown in figure 31 the lock member 370 is already in a distal end position as it encounters a proximal end of locking arms 322 of the needle cover sleeve 320. However, the plunger rod 340 is not yet completely inserted and as shown in figure 33 a sloped wall 349 of the first guiding cam 346 abuts a proximal edge 323 of the needle cover sleeve 320. The lock member clamping arms 371 engage the first recess 395 in the plunger rod as shown in figure 31.

In figure 33 it can be seen that a flange 375 of the lock member 370 is axially aligned with a locking recess 356 in the housing 350. In the position shown in figure 33 the flange 375 is not yet inserted into the housing recess 356.

When the drive unit 303 is further inserted into the syringe unit housing 350 the sloped wall 349 is further pressed against the edge 323 and subsequently the edge slides along the sloped wall 349. This causes the plunger rod 340 to rotate relative to the supporting member 360 and also relative to the housing 350.

Moreover, the distal movement of the plunger rod during insertion moves the plunger rod 340 relative to the lock member 370 as the latter abuts the syringe holder locking arms 322. Due to the relative movement the snap-fit connection between the clamping arms 371 and the first recess 395 (see figure 31) is released and the clamping arms 371 snap in the second recess 396 as shown in figure 32. The lock member 370 is thus rotationally and axially locked to the plunger rod 340.

Figures 32 and the corresponding perspective view in figure 34 show a final assembly state when the drive unit 303 is completely inserted into the housing 350. As it can be seen in figure 34 the lock member 370 is rotated together with the plunger rod 340 relative to the housing 350 into a final position. As depicted in figure 32 two oppositely arranged snap elements 363 of the supporting member are snaped in corresponding recesses 351 in the housing and thus the supporting member 360 is fixated to the housing 350 and cannot move relative thereto.

The above-mentioned rotation of the plunger rod 340 relative to the supporting member 360 during assembly causes the cams 366 of the supporting member 360 to get out of the radial section 343 of the plunger rod 340 (figures 29 and 30). Therefore, the bayonet connection is partly released and the sloped release surface 365 of the cam engages the sloped release surface 345 of the plunger rod. As the plunger rod 340 is biased by the injection spring 390 the two sloped surfaces 365, 345 are pressed against each but the plunger rod cannot rotate because it is held in place by a second guiding cam 347 which has entered a linear guide track 321 in the needle cover sleeve 320, see figure 32. The plunger rod 340 comprises two oppositely arranged second guiding cams 347 which are also shown in figure 28 and 29. The second guiding cams 247 are positioned 90° relative to the first guiding cams 270. Correspondingly, the needle cover sleeve 320 comprises two oppositely arranged linear guide tracks 321 also shown in figure 27. The biased plunger rod 340 is thus held in place and cannot move.

Furthermore, as shown in figure 34 due to the rotation of the plunger rod 340 the flange 375 of the lock member 370 is inserted into the locking recess 356 in the housing 350. That means the plunger rod 340 is thus axially locked to the housing by the lock member 370 such that the plunger rod cannot move along the longitudinal axis in this rotational orientation.

The assembled autoinjector 300 is then in a ready to use state. The cap 310 is mounted on the distal end and the securing arms 352 of the housing as shown in figure 32 prevent a proximal retraction movement of the needle cover sleeve 320 as described with respect to the first embodiment.

Before an injection the user has to remove the cap 310. The RNS 17 is removed together with the cap 310 if the use pulls off the cap as hooks of the cap engage a proximal rim of the RNS. When the cap 310 is removed from the needle cover sleeve 320 the securing arms 352 of the housing 350 are no longer prevented from being deflected radially inwards. That means if the user pushes the autoinjector against the injection site the needle cover sleeve 320 can move proximally thereby deflecting knob ends 353 (figure 32) of the securing arms 352. That means the securing arms 352 can deflect radially inwards to release the needle cover sleeve 320.

Therefore, the cover sleeve 320 moves thus from its covering position into its retracted position. This is enabled as cutouts 372 (see figure 27) in the lock member 370 are rotationally aligned with locking arms 322 of the needle cover sleeve such that the locking arms can pass the lock member 370.

During the proximal retraction movement, the second guiding cams 347 get out of the linear guide track 321 in the needle cover sleeve and enter into an opening or cavity 324 (figure 32 and 27). That means the plunger rod 340 is no longer axially guided. That in turn means the sloped release surfaces 345 of the plunger rod 340 which are pressed against the release surfaces 365 of the supporting member can slide along each other causing the plunger rod 340 to rotate relative to the supporting member 360. Thereby, the cam 366 gets into the axial groove section 342 in the guide track 341 and therefore the bayonet connection is released. The plunger rod 340 can thus be moved in dispensing direction by a force of the biased injection spring 390.

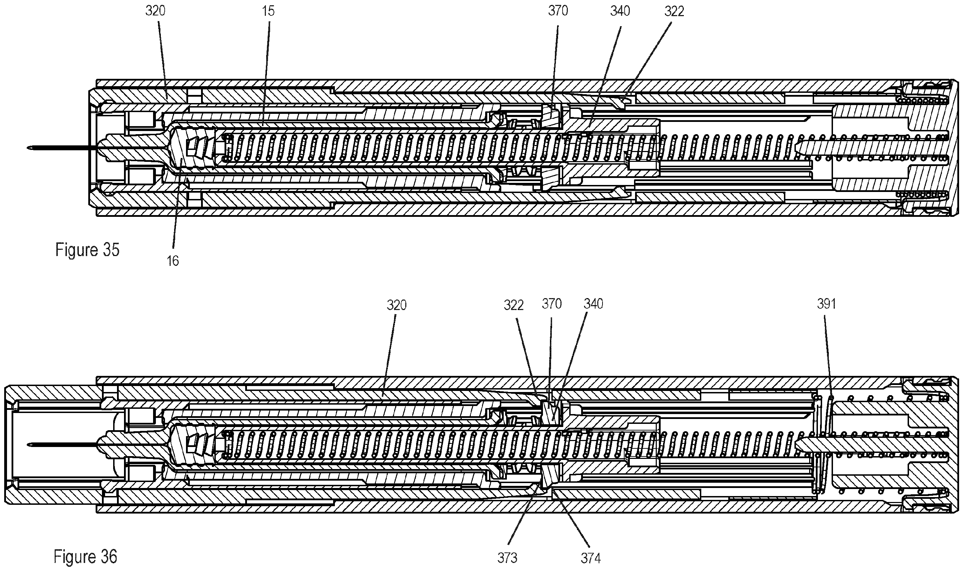

In the state shown in figure 35 the needle cover sleeve 320 is in its retracted position and the autoinjector 300 is in a state where the plunger rod 340 is in a distal end position and thus the piston 16 is in a distal end position and the drug is completely dispensed.

As the plunger rod 340 is rotated also the lock member 370 is rotated relative to the housing 350 and relative to the needle cover sleeve 320. That means after the needle cover sleeve is moved proximally and after rotation of the plunger rod the cutouts 372 in the flange 375 as shown in figure 27 for the locking arms 322 are no longer rotationally aligned with the locking arms.

Once the injection is completed the user removes the autoinjector 300 from the injection site. Due to the cover sleeve spring 391 the needle cover sleeve 320 is moved from the retracted position back into its distal covering position. This state of the autoinjector 300 is shown in figure 36.

As the plunger rod 340 has been rotated during needle cover retraction movement as described above the flange 375 of the lock member 370 is rotationally aligned with the locking arms 322. As shown in figure 36 the flange 375 has a stop surface 373 on a distal side and a ramp 374 on the proximal side. Before the needle cover sleeve 320 reaches its final covering position again the locking arms 322 are radially deflected outwards by the ramps 374 of the lock member 370 such that the locking arms 322 can pass and move in distal direction. As the flange 375 includes the stop surface 373 on the distal side the arms 322 encounter the stop surface 373 and thus the needle cover sleeve 320 is prevented from being moved towards the retracted position again. The needle cover sleeve 320 is thus locked in its covering position (needle cover lock).

The autoinjector according to the fourth embodiment may be disassembled and the parts made of the same material can be discarded of together. Alternatively, and as described with respect to the first embodiment the autoinjector may be reused after a reset procedure.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

### First embodiment: Figures 1 to 8

| | | | | | |
|---|---|---|---|---|---|
| 1 | autoinjector | 50 | housing | 80 | needle cover sleeve |
| 2 | syringe unit | 51 | snap element | 81 | recess |
| 3 | drive unit | 52 | securing arms | 84 | lock sleeve |
| | | 53 | knob end | 85 | locking arms |
| 10 | cap | 54 | sloped surface | 86 | cam end |
| 11 | remover sleeve | 55 | connecting portion | 87 | protrusion |
| 12 | hooks | | | 88 | first recess |
| 15 | prefilled syringe | 60 | supporting member | 89 | second recess |
| 16 | piston | 61 | sleeve-shaped portion | | |
| 17 | rigid needle shield RNS | 62 | guiding pin | 90 | injection spring |
| 18 | injection needle | 63 | end wall | 91 | cover sleeve spring |
| | | 64 | distal recess | 92 | release surface |
| 20 | needle cover sleeve | 65 | distal retaining arms | 93 | ramp |
| 21 | ledge | 66 | distal knob | 94 | recess |
| 22 | distal snap element | 67 | proximal retaining | 95 | rounded corner |
| 23 | securing rip | | arms | 96 | support surface |
| 24 | sloped surfaces | 68 | proximal knob | 97 | proximal recess |
| 25 | proximal snap element | 69 | sloped surface | | |
| 26 | inner surface | | | | |
| | | 70 | lock sleeve | | |
| 30 | syringe holder | 71 | ledge | | |
| 31 | rim | 72 | sloped surface | | |
| | | 75 | locking arms | | |
| 40 | plunger rod | 76 | cam end | | |
| 41 | distal recess | 77 | recess | | |
| 42 | proximal recess | 78 | holding arms | | |
| 43 | sloped surface | 79 | holding cams | | |
| 44 | release surface | | | | |

### Second embodiment: Figures 9 to 16

| | | | |
|---|---|---|---|
| 100 | autoinjector | 150 | housing |
| 102 | syringe unit | 151 | snap element |
| 103 | drive unit | 152 | recess |
| | | 153 | sloped contact surface |
| 110 | cap | | |
| 15 | prefilled syringe | 160 | supporting member |
| 16 | piston | 161 | sleeve-shaped portion |
| 17 | rigid needle shield RNS | 162 | guiding pin |
| 18 | injection needle | 163 | end wall |
| | | 164 | L-shaped snap element |
| 120 | needle cover sleeve | 165 | radial cam |
| 126 | U-shaped groove | 166 | radial snap element |
| 127 | angled wall | 167 | retaining arm |
| 128 | stop wall | 168 | protrusion |
| | | 169 | guiding cam |
| 130 | syringe holder | | |
| 131 | flange | 170 | spring element |
| 132 | nose | 171 | opening |
| 133 | nose | | |
| | | 190 | injection spring |
| 140 | plunger rod | 191 | cover sleeve spring |
| 141 | recess | 192 | nose |
| 142 | recess snap element | 193 | |
| 143 | holding ribs | | |
| 144 | end surface | | |
| 145 | arrowhead | | |
| 146 | connecting portion | | |

### Third embodiment: Figures 17 to 26

| | | | |
|---|---|---|---|
| 200 | autoinjector | 250 | housing |
| 202 | syringe unit | 251 | recess |
| 203 | drive unit | 252 | recess |
| | | 255 | connecting portion |
| 210 | cap | | |
| 211 | tongues | | |
| 212 | hooks | 260 | supporting member |
| 15 | prefilled syringe | 261 | sleeve-shaped portion |
| 16 | piston | 262 | guiding pin |
| 17 | rigid needle shield RNS | 263 | end wall |
| 18 | injection needle | 264 | snap arm |
| | | 265 | retaining arms |
| 220 | needle cover sleeve | 266 | protrusion |
| 221 | extension | 267 | cam |
| | | 268 | angled wall |
| 230 | syringe holder | 269 | axial rib |
| 231 | cam | | |
| | | 270 | lock sleeve |
| 240 | plunger rod | 271 | retaining nose |
| 241 | recess | 272 | sloped surface |
| 242 | sloped surface | 273 | stop surface |
| 245 | plunger rod | 274 | locking surface |
| 246 | saw teeth | 276 | first axial groove |
| 247 | plunger rod | 277 | second axial groove |
| 248 | guiding pin | 278 | angled section |
| | | 279 | sawtooth |
| | | 280 | spring element |
| | | 281 | opening |
| | | 285 | locking arms |
| | | 290 | injection spring |
| | | 291 | cover sleeve spring |
| | | 292 | deflectable arm |
| | | 293 | nose |
| | | 294 | supporting member |
| | | 295 | supporting member |
| | | 296 | deflectable arm |
| | | 297 | nose |

### Fourth embodiment: Figures 27 to 36

| | | | |
|---|---|---|---|
| 300 | autoinjector | 350 | housing |
| 302 | syringe unit | 351 | recess |
| 303 | drive unit | 352 | securing arms |
| | | 353 | knob end |
| 15 | prefilled syringe | 355 | connecting portion |
| 16 | piston | 356 | locking recess |
| 17 | rigid needle shield RNS | | |
| 18 | injection needle | 360 | supporting member |
| | | 361 | sleeve-shaped portion |
| 310 | cap | 362 | guiding pin |
| 320 | needle cover sleeve | 363 | snap element |
| 321 | linear guide track | 364 | lock surface |
| 322 | locking arms | 365 | release surface |
| 323 | edge | 366 | cam |
| 324 | cavity | | |
| | | 370 | lock member |
| 330 | syringe holder | 371 | clamping arms |
| 331 | rim | 372 | cutout |
| | | 373 | stop surface |
| 340 | plunger rod | 374 | ramp |
| 341 | guide track | 375 | flange |
| 342 | axial groove section | | |
| 343 | radial section | 390 | injection spring |
| 344 | lock surface | 391 | cover sleeve spring |
| 345 | release surface | | |
| 346 | first guiding cam | 395 | first recess |
| 347 | second guiding cam | 396 | second recess |
| 349 | sloped wall | | |

## Claims

1. Injection device (1) for dispensing a liquid drug from a syringe (15) through an injection needle (18), the injection device (1) comprising a pre-assembled drive unit (3) connected to a pre-assembled syringe unit (2), wherein the pre-assembled syringe unit (2) comprises
- a syringe holder (30) defining a longitudinal axis;
- a needle cover (20) movable along the longitudinal axis relative to the syringe holder between a needle covering position and a retracted position in which the needle (18) protrudes from the needle cover (20),
and wherein the pre-assembled drive unit (3) comprises
- a plunger rod (40);
- a spring member (90) adapted bias the plunger rod (40) in a dispensing direction;
- a supporting member (60) adapted to support or guide the spring member (90) or the plunger rod (40);
- a first and a second retaining element (67, 65), wherein each retaining element (67, 65) can be in a holding position in which the first or second retaining element holds the biased plunger rod (40) in place relative to the supporting member (60) and in a release position in which the first or second retaining element (67, 65) does not hold the biased plunger rod (40) in place,
wherein prior to assembly of the injection device (1) the pre-assembled drive unit (3) and the pre-assembled syringe unit (2) are separated from each other, and the first retaining element (67) is in its holding position and the biased plunger rod (40) is exclusively held in place by the first retaining element (67);
wherein during connection of the syringe unit (2) to the drive unit (3) a movement of the needle cover (20) relative to the supporting member (60) causes the first retaining element (67) to move out of its holding position,
wherein when the injection device (1) is completely assembled the drive unit (3) is connected to the syringe unit (2) and the needle cover (20) is operatively coupled to the second retaining element (65) wherein
- the first retaining element (67) is in its release position and
- the second retaining element (65) is in its holding position such that the biased plunger rod (40) is exclusively held in place by the second retaining element (65) when the needle cover (20) is in its covering position.

2. Injection device (1) according to claim one, wherein in the assembled state of the injection device the second retaining element (65) is in its release position when the needle cover (20) is in its retracted position.

3. Injection device (1) according to claim 1 or 2, wherein the supporting member (60) comprises the further comprises the first and a second retaining element (67, 65), and wherein the supporting member (60) comprises at least one of the following
- a connecting element (94) adapted to connect the pre-assembled drive unit (3) to the pre-assembled syringe unit (2);
- a guiding element (95) adapted to guide the plunger rod along the longitudinal direction;
- a support surface (96) for supporting the spring member at a proximal end of the spring member.

4. Injection device (1) according to any of claims 1 to 3, wherein the supporting member (60) further includes a sleeve-shaped portion (61) comprising a circumferential wall defining a bore, wherein the biased plunger rod is at least partly arranged inside the bore.

5. Injection device (1) according to claim 4, wherein the first and second retaining element (67, 65) are arranged in the wall.

6. Injection device (1) according to any of claims 1 to 5, wherein the first retaining element (67) is a deflectable arm extending in the longitudinal direction with a retaining portion (68) on its free end.

7. Injection device (1) according to any of claims 1 to 6, wherein the plunger rod (40) is hollow defining a cavity and wherein the spring member (90) is arranged inside the cavity and wherein the supporting member (60) comprises a guiding pin (62) extending along the longitudinal axis inside the spring member (90) and inside the cavity of the hollow plunger rod (40) to guide the spring member (90).

8. Injection device (1) according to any of claims 1 to 7, wherein the pre-assembled syringe unit (2) further comprises a sleeve-shaped housing (50) and wherein the needle cover (20) is coaxially arranged inside the housing (50) and the syringe holder (30) is arranged inside the needle cover (20).

9. Injection device (100) according to claim 8, wherein the housing (150) comprises a release surface (153) adapted to move the first retaining element (167) out of its holding position and into its release position during connection of the pre-assembled syringe unit (102) with the pre-assembled drive unit (103).

10. Injection device (1) according to claim 8 or 9, wherein in an assembled state proximally to the syringe holder (30) the housing comprises a connecting portion (55) which extends along the longitudinal axis at least a half, preferably a full length of the axial length of the syringe holder (30) and wherein the connecting portion (55) is adapted to accommodate and to be connected to the drive unit (3).

11. Injection device (1) according to any of claims 1 to 10, wherein the drive unit (3) further comprises a lock sleeve (70) movable relative to the supporting member (60) and adapted to lock, in the assembled state, the second retaining element (65) in its holding position such that the second retaining element (65) cannot be brought into its release position.

12. Injection device (100) according to any of claims 1 to 10, wherein the supporting member (160) comprises an elastic element (170) on its distal end adapted to bias the syringe inside the syringe holder in an assembled state of the injection device, wherein the elastic element (170) is integrally formed in the supporting member (160) as a monolithic part.

13. Injection device (1) according to any of claims 1 to 12, wherein the supporting member (60), the first and second retaining element (67, 65) and the plunger rod (40) are made of the same material, preferably polyoxymethylene.

14. Injection device (1) according to any of claims 1 to 13, wherein the syringe holder (30) and the needle cover (20) are made of the same material, preferably polypropylene.

15. Method for assembly an injection device (1) comprising the steps of
a) Inserting a distal portion of a pre-assembled drive unit (3) according to any of claims 1 to 14 into the connecting portion (55) of the housing (50) of a pre-assembled syringe unit (2) according to any of claims 1 to 14 and thereby releasing the first retaining element (67) from the plunger rod (40) and subsequently holding the plunger rod by the second retaining element (65);
b) Connecting the supporting member (60) to the housing (50) by the connecting element to establish a snap-fit connection between the supporting member and the housing.
